# EUROPEAN PATENT APPLICATION

(11) **EP 2 298 782 A1**
(43) Date of publication of application: **23.03.2011**
(21) Application number: 09290651.0
(22) Date of filing: 26.08.2009
(51) Int. Cl.: C07H 1/00, C07H 17/02

(54) **Method for producing pyrazole glycoside derivatives**

(71) Applicant: Sanofi-Aventis, 75013 Paris (FR)
(72) Inventor: The designation of the inventor has not yet been filed
(74) Representative: Fischer, Hans-Jürgen

(57) **Abstract**

A process for preparing pyrazole-glycoside derivatives of the general formula (I) 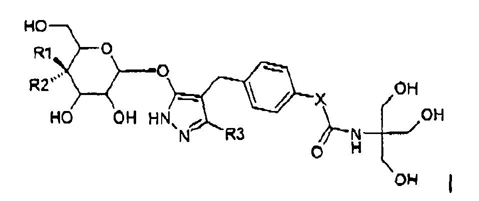 in which the meanings are
R1
H and R2 F; or
R1
F and R2 H; or
R1
F and R2 F;
R3
(C₁-C₈)-alkyl, where one, more than one or all hydrogen(s) may be replaced by fluorine;
X
(C₁-C₃)-alkylene, (C₂-G₃)-alkenylone.

## Description

The present invention relates to a process for preparing pyrazole-glycoside derivatives of the general formula (I)

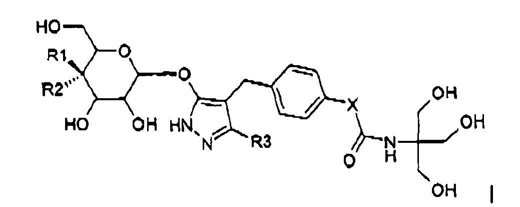

WO20051121161 describes inter alia various processes for preparing the pyrazole - glycoside derivative of the formula (la).

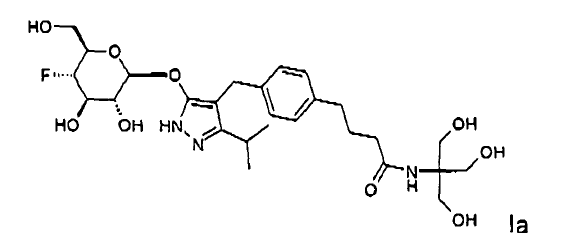

These pyrazole-glycoside derivatives show biological activity which makes their use possible in particular in the prevention and treatment of type 1 and 2 diabetes.

However, the late introduction of the butanoic side chain by Heck-coupling with vinyl acetate leads to undesired regioisomers which have to be separated by sophisticated HPLC methods. The lowering in yield and the effort for separation of these regioisomers makes this synthesis route not viable for a production in industrial scale.

In view of the disadvantages and problems described above, there is a need to provide a process which avoids these disadvantages and problems and which moreover, without requiring great additional complexity, can be implemented in a simple manner and makes the desired products available in high yields with high conversion and high selectivity. High yields in particular are a central requirement for the process which is sought.

This object is surprisingly achieved by a process for preparing compounds of the formula (I): 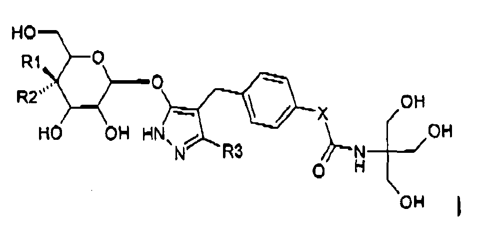 in which the meanings are
- R1: H and R2 F; or
- R1: F and R2 H; or
- R1: F and R2 F;
- R3: (C₁-C₈)-alkyl, where one, more than one or all hydrogen(s) may be replaced by fluorine;
- X: (C₁-C₃)-alkylen.e, (C₂-C₃)-alkenylene;
which comprises applying a multistage process in which

### A. Preparation of the beta-keto-ester

### A.1. the component of the formula (II)

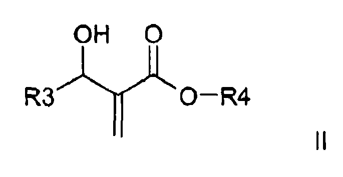

in which
- R3: is (C₁-C₈)-alkyl, where one, more than one or all hydrogen(s) may be replaced by fluorine; preferably methyl, ethyl, propyl, isopropyl or t-butyl;
- R4: is (C₁-C₈)-alkyl, preferably methyl, ethyl, propyl, isopropyl or t-butyl;
is reacted with 0.5 to 2 equivalents, preferably 0.8 to 1.2 equivalents of compound of the formula (III) 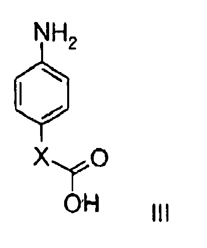 in which
- X: is (C₁-C₃)-alkylene, (C₂-C₃)-alkenylene;
by first treating compound of formula III in the presence of from 0.1 to 10 equivalents, preferably 0.8 to 1.5 equivalents, of one or more acids - where one acid is preferred - preferably with acids selcted from CF₃SO₃H, H₂SO₄, toluenesulfonic acid, HBF₄ or HPF₆, particularly preferably with acids selected from H₂SO₄, HBF₄ or HPF₆, in a suitable solvent, preferably in water, at from -50°C to 0°C, preferably at from -20°C to 0 °C, particularly preferably at from -5°C to 0°C with 1.0 to 1.5 equivalents of NaNO₂.
and by adding this mixture to a mixture of 0.8 to 1.5 equivalents, preferably 0.9 to 1.1 equivalents of the component of formula II comprising a catalyst, preferably Pd-catalyst, more preferably Pd-II-acetate, as a salt or in the presence of C-black to facilitate the subsequent Pd removal, or Pd on carbon, in a suitable solvent, which is watermiscible, preferably acetonitrile, at from 0°C to 100°C, preferably at from 0°C to 80 °C, more preferably from 20°C to 60°C;
to give a compound of the formula (IV), 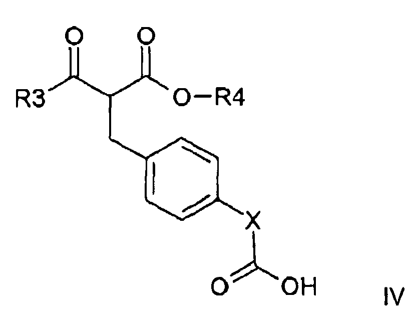
in which X, R3 and R4 are as defined above;
and
optionally the compound of the formula (IV) is purified by conventional purification methods such as crystallization, distillation or chromatography, preferably by crystallization from a solvent or a mixture of a plurality of solvents such as alkanes, aromatic compounds, halogenated solvents, ethers, ketones, esters, alcohols or water, particularly preferably purified by crystallization from methanol or from dichloromethane/heptane or methanol/water mixtures or by sodium salt and - after neutralization - crystallization from water;

### B. Preparation of pyrazolones

### B.1. A compound of formula IV

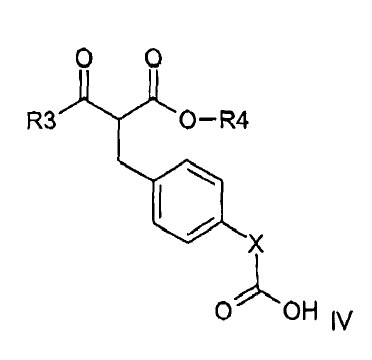

in which X, R3 and R4 are as defined above;
is converted to a compound of formula V 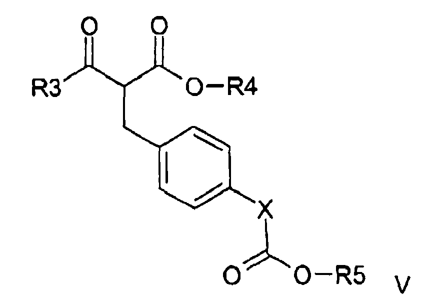
where R5 is is (C₁-C₆)-alkyl and X. R3 and R4 are as defined above;
by treatment with a lower alcohol R5-OH in the presence of an acidic catalyst and a water removing reagent, preferably EtOH or MeOH in the presence of SOCl₂;
and the compound of formula V is subsequent reacted with 0.8 to 1.5 equivalents, preferably 0.9 to 1.1 equivalents the compound 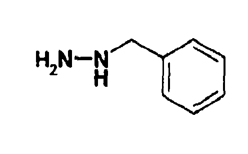 in the presence of an acidic catalyst wherein an example is the use of toluenesulfonic acid; further suitable acidic catalysts are organic acids, preferably acetic acid or propionic acid;
if the hydrazine is used in the form of a salt, addition of 0.5 to 2 equivalents of a base can speed up the reaction; suitable bases are the alkali salts of the respective carboxylic acids, such as sodium acetate or potassium propionate. It is apparent that these salts can be generated in situ by the addition of NaOH, KOH or NaOMe or KOMe to the carboxylic acid;
at from -50°C to +150°C, preferably at from -20°C to +100°C, particularly preferably at from 60°C to 75°C,
to give a compound of the formula (VI) 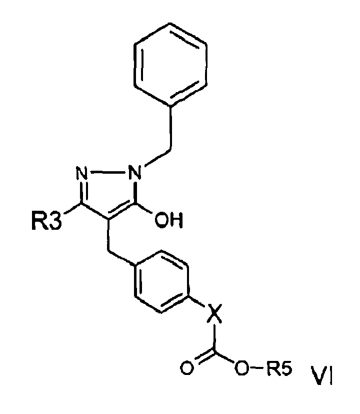
in which X, R3 and R5 are as defined above;
   and
   optionally the compound of the formula (VI) is purified by conventional purification methods such as crystallization, distillation or chromatography, preferably by crystallization from a solvent or a mixture of a plurality of solvents such as alkanes, aromatic compounds, halogenated solvents, ethers, ketones, esters, alcohols or water, particularly preferably purified by crystallization from methanol or from dichloromethane/heptane or methanol/water mixtures or by sodium salt and - after neutralization - crystallization from water;
or

### B.2. A compound of formula IV

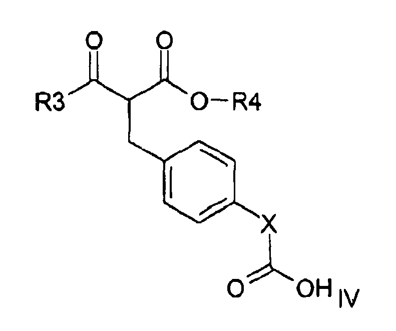

in which X, R3 and R4 are as defined above.
is reacted reacted with 0.8 to 1.5 equivalents , preferably 0.9 to 1.1 equivalents of the compound 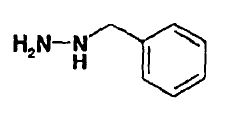 in the presence of an acidic catalyst, wherein an example is the use of toluenesulfonic acid; further suitable acidic catalysts are organic acids, preferably acetic acid or propionic acid;
if the hydrazine is used in the form of a salt, addition of 0.5 to 2 equivalents of a base can speed up the reaction; suitable bases are the alkali salts of the respective carboxylic acids, such as sodium acetate or potassium propionate;
at from -50°C to +150°C, preferably at from -20°C to +100°C, particularly preferably at from 60°C to 75°C,
to give a compound of formula Via 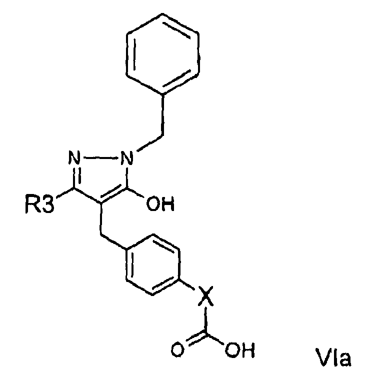
in which X and R3 are as defined above;
which is reacted further by treatment with a lower alcohol R5-OH in the presence of an acidic catalyst and a water removing reagent, preferably EtOH or MeOH in the presence of SOCl₂;
to give a compound of formula VI 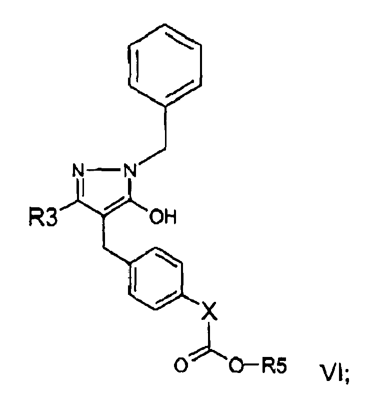 and
optionally the compound of the formula (VI) is purified by conventional purification methods such as crystallization, distillation or chromatography, preferably by crystallization from a solvent or a mixture of a plurality of solvents such as alkanes, aromatic compounds, halogenated solvents, ethers, ketones, esters, alcohols or water, particularly preferably purified by crystallization from methanol or from dichloromethane/heptane or methanol/water mixtures or by sodium salt and - after neutralization - crystallization from water;

### C. Preparation of pyrazolone glycoside

### C.1. A compound of the formula (VI)

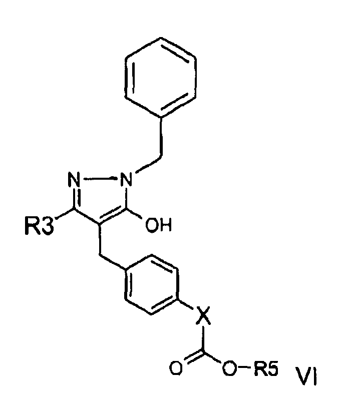

is reacted with a sugar derivative of the formula (VII) 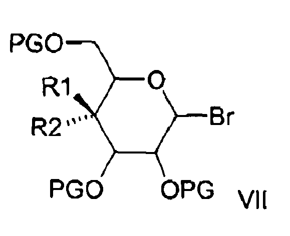 in which
R1 and R2 are defined as described above;
PG is an OH protective group such as, for example, methyl, methoxymethyl (MOM), methylthiomethyl (MTM), phenyldimethylsilylmethoxymethyl (SMOM), benzyloxymethyl (BOM), p-methoxybenzyloxymethyl (PMBM), t-butoxymethyl, 4-pentenyloxymethyl, 2-methoxyethoxymethyl (MEM), 2-trimethylsilylethoxymethyl (SEM), trimethylsilyl (TMS), tert-butyldimethylsilyl (TBDMS), tert-butyldiphenylsilyl (TBDPS), triisopropylsilyl (TIPS), or similar silyl protective groups, 1-methyl-1-methoxyethyl (MIP), allyl, benzoyl, acetyl, trifluoroacetyl, Fmoc, THP, and preferably acetyl or benzoyl;
   by adding 0.95 to 1.2 equivalents of a Li base to a compound of formula VI in an ethereal solvent, wherein suitable ethereal solvents are 1,2-dimethoxy ethane, 1,2-diethoxy ethane, 1,4-dioxane, tetrahydrofuran, methyl-tetrahydrofuran or cyclopentyl methyl ether, preferred is tetrahydrofuran, 1,4-dioxane or methyl tetrahydrofuran, more preferred tetrahydrofuran or methyl tetrahydrafuran, wherein suitable bases are LiH, alkyl lithium reagents, aryl lithium reagents or Li alkoxides, preferred are Li alkoxides, more preferred are Li alkoxides derived from hindered tertiary alcohols, such as Li tert-butoxide;
and the Li salt of the compound of the formula (VI) is subsequently reacted with 0.5 to 2 equivalents the compound of the formula VII, preferably 0.9 to 1.1 equivalents, more preferred 0.9 to 1 equivalents, at 40°C to 120°C, preferably of 60°C to 100°C; to give the compound of the formula (VIII); 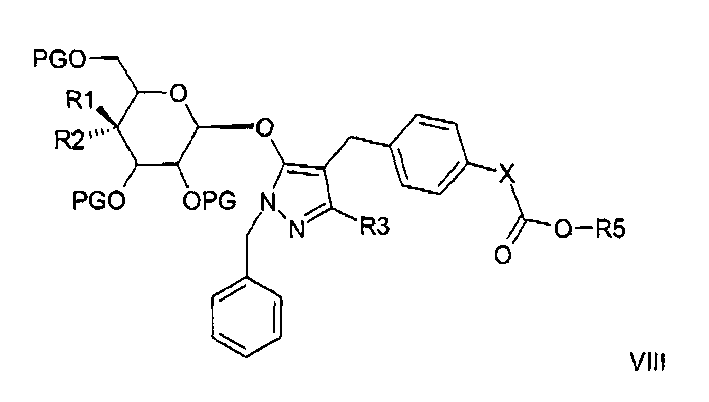
in which PG, X, R1, R2, R3 and R5 are as defined above;
   and
   optionally the compound of the formula (VIII) is purified by conventional purification methods such as crystallization, distillation or chromatography, preferably by crystallization from a solvent or a mixture of a plurality of solvents such as alkanes, aromatic compounds, halogenated solvents, ethers, ketones, esters, alcohols or water, particularly preferably purified by crystallization from methanol or from dichloromethane/heptane or methanol/water mixtures or by sodium salt and - after neutralization - crystallization from water;
or

### C.2. the compound of the formula (VI)

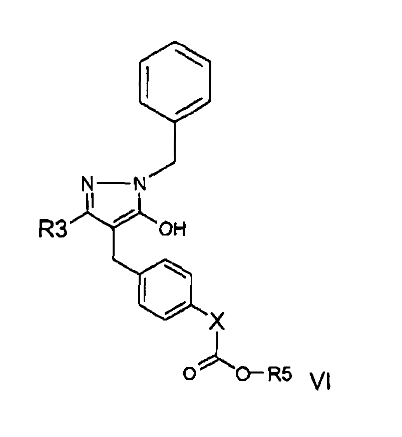

is reacted with 0.5 to 2 equivalents the compound of the formula VII, preferably 0.9 to 1.1 equivalents, more preferred 0.9 to 1 equivalents, of a sugar derivative of the formula (VII) 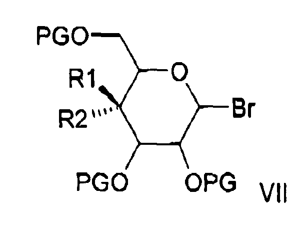 in which
R1 and R2 are defined as described above;
PG is an OH protective group such as, for example, methyl, methoxymethyl (MOM), methylthiomethyl (MTM), phenyldimethylsilylmethoxymethyl (SMOM), benzyloxymethyl (BOM), p-methoxybenzyloxymethyl (PMBM), t-butoxymethyl, 4-pentenyloxymethyl, 2-methoxyethoxymethyl (MEM), 2-trimethylsilylethoxymethyl (SEM), trimethylsilyl (TMS), tert-butyldimethylsilyl (TBDMS), tert-butyldiphenylsilyl (TBDPS), triisopropylsilyl (TIPS), or similar silyl protective groups, 1-methyl-1-methoxyethyl (MIP), allyl, benzoyl, acetyl, trifluoroacetyl, Fmoc, THP, and preferably acetyl or benzoyl;
in an inert solvent in the presence of a trialkyl amine base, wherein suitable solvents are tetrahydrofuran, methyl tetrahydrofuran, 1,4-dioxane, acetonitrile, propionitrile or N-methylpyrrolidinone, preferred is tetrahydrofuran or acetontrile, with acetonitrile being more preferred, wherein suitable trialkylamine bases are triethylamine, isopropyldiethylamine, diisopropylethylamine, tributylamine, benzyldiethylamine or trioctylamine, preferred is triethylamine, tributylamine, trioctylamine or diisopropylethylamine, with triethylamine and diisopropylethylamine being more preferred;
at 40°C to 120°C, preferably of 60°C to 100°C;
to give a compound of formula VIII 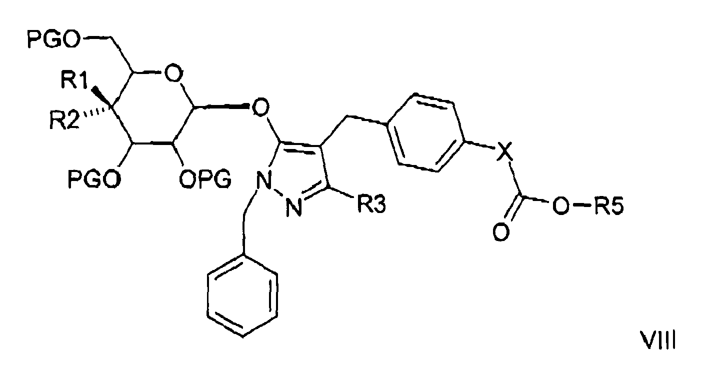
in which PG, X, R1, R2, R3 and R5 are as defined above;
   and
   optionally the compound of the formula (VIII) is purified by conventional purification methods such as crystallization, distillation or chromatography, preferably by crystallization from a solvent or a mixture of a plurality of solvents such as alkanes, aromatic compounds, halogenated solvents, ethers, ketones, esters, alcohols or water, particularly preferably purified by crystallization from methanol or from dichloromethane/heptane or methanol/water mixtures or by sodium salt and - after neutralization - crystallization from water;

### D. Preparation of the pyrazole-glycoside derivative

### D.1. The compound of formula VIII

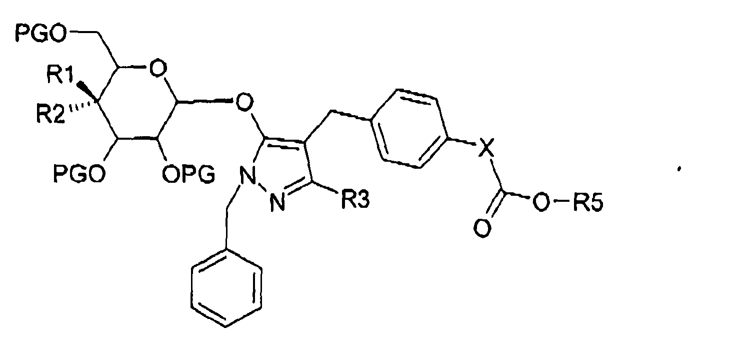

in which PG, X, R1, R2, R3 and R5 are as defined above;
is converted to compound IX 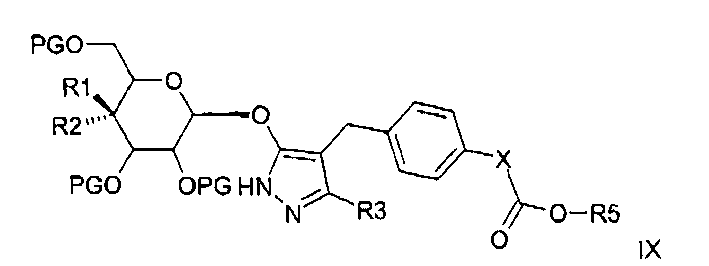
in which PG, X, R1, R2, R3 and R5 are as defined above;
by hydrogenolysis with a transition metal catalyst, preferably in hetereogenous form deposited on a solid support carrier, more preferred is Pd or Rh deposited on C, particulary preferred is Pd/C, wherein suitable solvents are lower alcohols or esters, preferably MeOH, EtOH, propanol, ethyl acetate or isopropyl acetate at +10°C to +80°C at a hydrogen pressure of 1 bar to 60 bars;
and optionally the compound of formula IX can be isolated using techniques known to those skilled in the art, but it can also be used in crude, unisolated form;

And the compound of the formula IX, where the definition of PG includes base sensitive protecting groups, such as acetate or benzoate, is subsequent converted to a compound of formula X 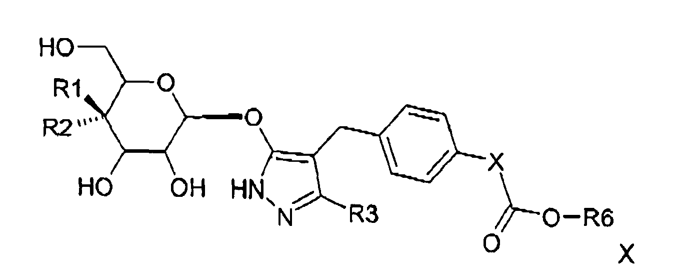
in which X, R1, R2 and R3 are as defined above;
R6 is (C₁-C₆)-alkyl, preferably methyl or ethyl;
   by reaction with an excess of a salt of an alcohol in alcoholic solvent wherein referred is a lower alcohol, more preferred a lower primary alcohols, most preferred is MeOH or EtOH, wherein suitably salts are alkali or earthalkali metal salts, preferably Li, Na, K or Mg, with Na or K being more preferred;
   preferred are 3 equivalents of alcoxide base, more preferred are 3 to 3.5 equivalents of NaOMe or KOMe in MeOH;
   at +10°C to +90°C, with +20°C to +60°C being more preferred;
   (As a consequence of this deprotection the ester R5 is converted largely to the alcohol used in this transformation. If MeOH is used, R6 will be largely Me, for EtOH R6 will be Ethyl);
and subsequent reacting an aqueous solution of the compound of formula X with an excess of a concentrated aqueous solution of tris(hydroxymethyl)aminomethane to give the compound of formula I 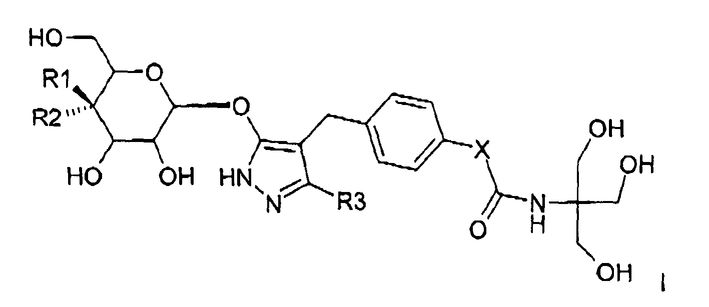
in which X, R1, R2 and R3 are as defined above;
wherein preferred is the use of 5 to 50 equivalents of tris(hydroxymethyl)aminomethane, with 10 to 20 equivalents being more preferred.
and subsequent the compound of the formula (I) is purified by conventional purification methods such as crystallization or chromatography, preferably by crystallization from a solvent or a mixture of a plurality of solvents such as alkanes, aromatic compounds, halogenated solvents, ethers, ketones, esters, alcohols or water, particularly preferably by crystallization from alcohols or alcohols/water mixtures, very particularly preferably by crystallization from methanol/water.

Or

### D.2. a compound of formula VIII

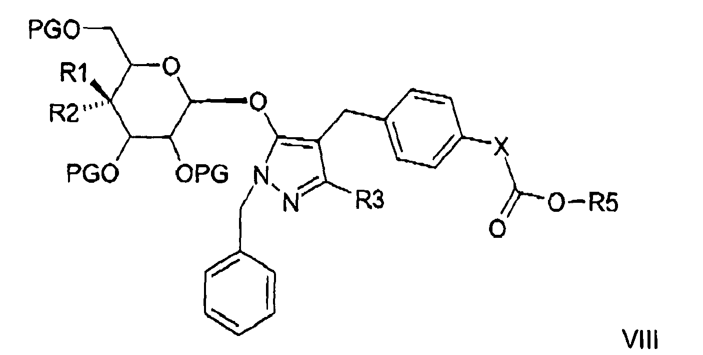

in which PG, X, R1, R2, R3 and R5 are as defined above;
is reacted with an excess tris(hydroxymethyl)aminomethane in an alcoholic solvent in the presence of an alcoxide, wherein suitable alcoxides are all alkali or earth alkali alcoxides, preferred are those of Li, Na, K or Mg, with Na or K being more preferred, wherein a suitable solvent is any alcohol, preferred is a tertiary alcohol, more preferred tert-butanol or tert-amylalcohol, most preferred is the use of K OtBu in tert-butanol;
preferable is the use of 5 to 50 equivalents of tris(hydroxymethyl)aminomethane, with 10 to 20 equivalents being more preferred,
to give a compound of formula XI 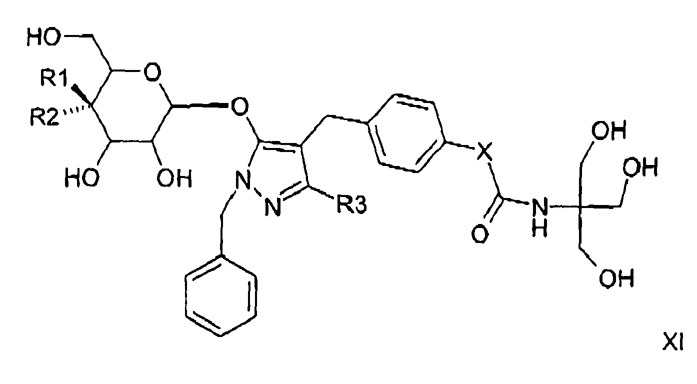
in which X, R1, R2 and R3 are as defined above;
and
subsequent hydrogenolysis with a heterogeneous platinum metal catalyst, preferably Pd or Rh deposited on a solid support, wherein a preferred solid support is C with Pd; at a hydrogen pressure of 1 bar to 60 bars, with 1 bar to 20 bars being preferred, more preferred is a pressure of 5 bars to 10 bars;
at 20°C to 90°C, with 30°C to 50°C being more preferred,
to give a compound of formula I 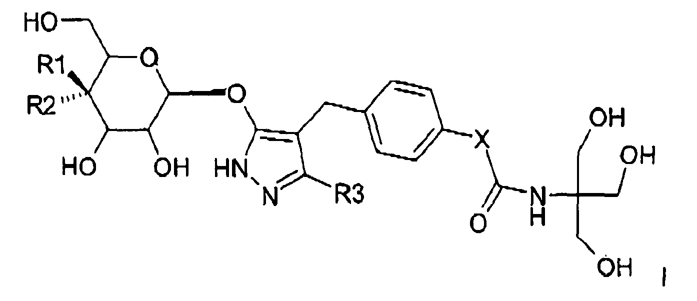
in which X, R1, R2 and R3 are as defined above;
and subsequently the compound of the formula (I) is purified by conventional purification methods such as crystallization or chromatography, preferably by crystallization from a solvent or a mixture of a plurality of solvents such as alkanes, aromatic compounds, halogenated solvents, ethers, ketones, esters, alcohol or water, particularly preferably by crystallization from alcohols or alcohols/water mixtures, very particularly preferably by crystallization from methanol/water.

The synthesis of compounds of formula II is described for example in W. Adam et al, JOC (1991), 56(20), 5782-5 and is the prototypical product of the Baylis-Hillman reaction.

The synthesis of F-sugar derivatives of formula VII is described for example in C.S. Rye, S.G. Withers, JACS (2002), 124(33), 9756-9767; or P.J, Card, JOC (1983), 48(3), 393-5; or in WO2004/052903.

In an alternative embodiment the process step A. has the following meaning

### A.2. the component of the formula (Ila)

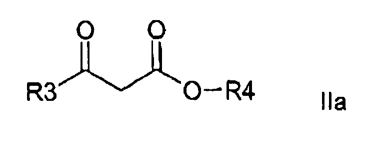

in which
   - R3: is (C₁-C₆)-alkyl, where one, more than one or all hydrogen(s) may be replaced by fluorine; preferably methyl, ethyl, propyl, isopropyl or t-butyl;
   - R4: is (C₁-C₆)-alkyl, preferably methyl, ethyl, propyl, isopropyl or t-butyl;
   is reacted with 0.5 to 2 equivalents, preferably 0.8 to 1.2 equivalents of compound of the formula (IIIa) 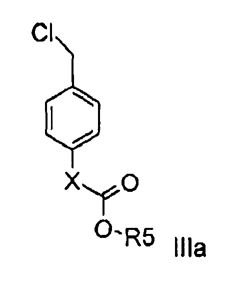 in which
   - X: is (C₁-C₃)-alkylene, (C₂-C₃)-alkenylene;
   - R5: is (C₁-C₆)-alkyl;
   in the presence of from 0.1 to 10 equivalents, preferably 0.8 to 1.5 equivalents, of a strong non-nucleophic base, optionally in the presence of a phase transfer catalyst, with 0.05 to 0.5 equivalents of a phase transfer catalyst, wherein suitable phase transfer catalysts are tetraalkylammonium halides, preferably tetrabutylammonium iodide, in a suitable solvent, wherein suitable non-nuclephilic bases are the alkali
   metal hydrides, preferably NaH. wherein a suitable solvent is an ethereal solvent, preferably THF, methyl-THF, 1,4-dioxane or 1,2-dimethoxyethane;
   at from -50°C to 50°C, preferably at from -20°C to 30 °C, particularly preferably at from -5°C to 5°C;
   to give a compound of the formula (V),

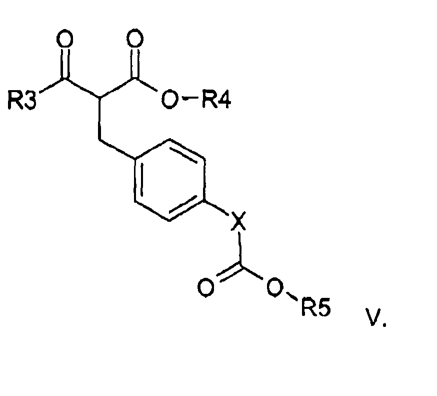

In an alternative embodiment the process step D. has the following meaning

### D. Preparation of the pyrazole-glycoside derivative

### D.3 The compound of formula VIII

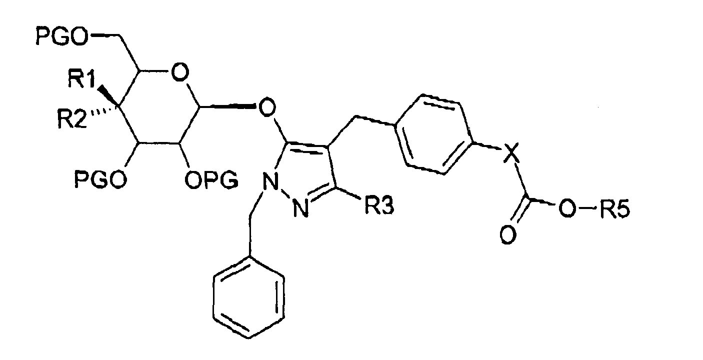

in which PG, X, R1, R2, R3 and R5 are as defined above;
is converted to compound IX 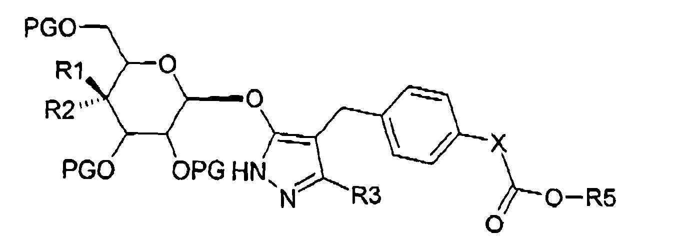
in which PG, X, R1, R2, R3 and R5 are as defined above;
by hydrogenolysis with a transition metal catalyst, preferably in hetereogenous form deposited on a solid support carrier, more preferred is Pd or Rh deposited on C, particulary preferred is Pd/C, wherein suitable solvents are lower alcohols or esters, preferably MeOH, EtOH, propanol, ethyl acetate or isopropyl acetate at +10°C to +80°C at a hydrogen pressure of 1 bar to 60 bars;
and optionally the compound of formula IX can be isolated using techniques known to those skilled in the art, but it can also be used in crude, unisolated form;
and the compound of the formula IX is subsequent converted to a compound of formula XII 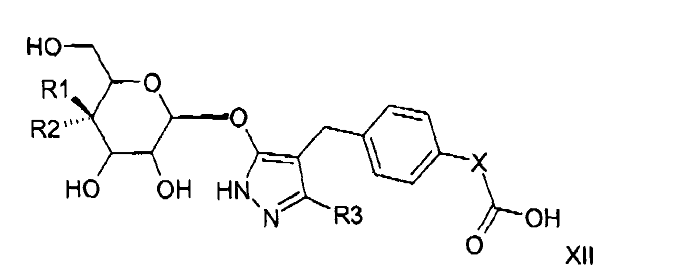
in which X, R1, R2 and R3 are as defined above;
by reaction with a base, preferably are 1 to 2 equivalents of base, more preferred are 1.1 to 1.2 equivalents, in an aqueous solvent, preferably water, wherein preferred bases are alkali or earthalkali hydroxides, more preferred is NaOH or KOH,
at +10°C to +90°C. preferably at +20°C to +60°C;
and subsequent reacting with an excess of tris(hydroxymethyl)aminomethane in a suitable solvent with an amide forming reagent to an solution or suspension of the compound of formula XII
to give the compound of formula I 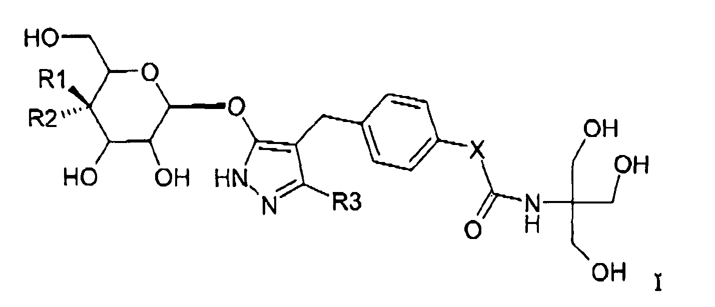
in which X, R1, R2 and R3 are as defined above;
wherein suitable solvents for this reaction are dipolar aprotic solvents, such as DMF, NMP or DMPU, wherein the amide forming reagents are known to those skilled in the art and may be added to the mixture of XII and tris(hydroxymethyl)aminomethane or alternatively, it is possible to add the amide forming reagent first to the compound of the formula XII before adding tris(hydroxymethyl)arninomethane., wherein suitable amide forming reagents are carbodiimides or CDI or 2-ethoxy-1-ethoxycarbonyl-1,2-dihydrochinolin, preferably 2-ethoxy-1-ethoxycarbonyl-1,2-dihydrochinolin;
and subsequent
isolation of the product is achieved by conventional methods like resin chromatography of the crude reaction mixture.
Or

### D.4. a compound of formula VIII

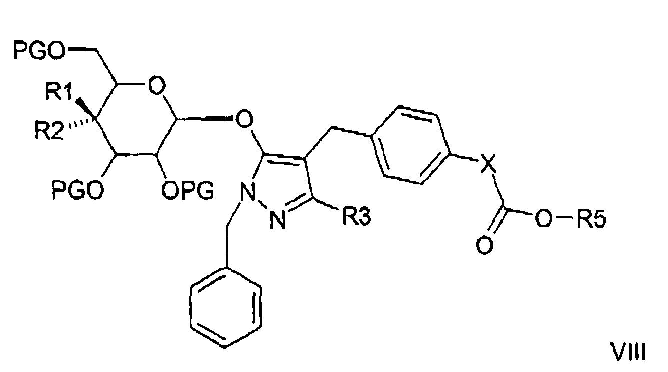

in which PG, X, R1, R2, R3 and R5 are as defined above;
is converted to the compound of the formula XIII 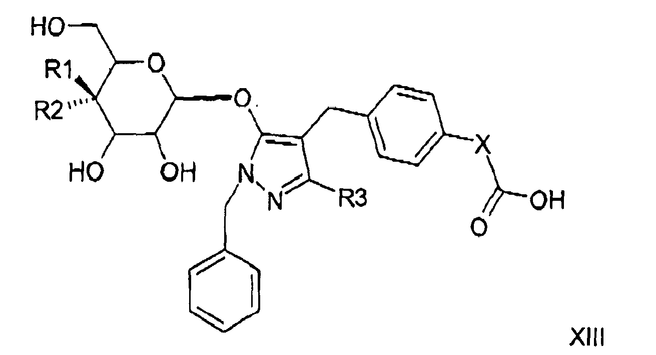
in which X, R1, R2 and R3 are as defined above;
by reaction with a base, preferably 1.0 to 2.0 equivalents of base, more preferred 1.1 to 1.2 equivalents, in an aqueous solvent, preferably water, wherein preferred bases are alkali or earthalkali hydroxides, more preferred are NaOH or KOH;
at +10°C to +90°C, preferably at +20°C to +60°C ;
and subsequent reacting of an solution or suspension of the compound of formula XIII with an excess of tris(hydroxymethyl)aminomethane in a suitable solvent with an amide forming reagent
to give a compound of formula XI 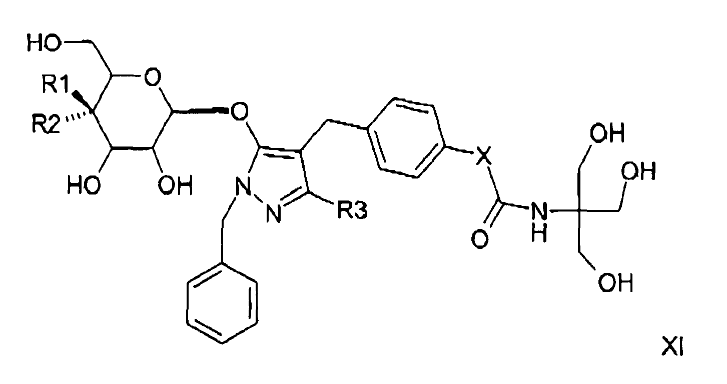
in which X, R1, R2 and R3 are as defined above;
wherein suitable solvents for this reaction are dipolar aprotic solvents, such as DMF, NMP or DMPU, wherein the amide forming reagents are known to those skilled in the art and may be added to the mixture of XIII and tris(hydroxymethyl)aminomethane or alternatively, it is possible to add the amide forming reagent first to the compound of the formula XIII before adding tris(hydroxymethyl)aminomethane, wherein suitable amide forming reagents are carbodiimides or CDI or 2-ethoxy-1-ethoxycarbonyl-1,2-dihydrochinolin, preferably 2-ethoxy-1-ethoxycarbonyl-1,2-dihydrochinolin;
and
subsequent hydrogenolysis with a heterogeneous platinum metal catalyst, preferably Pd or Rh deposited on a solid support, wherein a preferred solid support is C, with Pd/C being more preferred; at a hydrogen pressure of 1 bar to 60 bars, preferably 1 bar to 20 bars, more preferred is a pressure of 5 bars to 10 bars,
at 20°C to 90°C, preferably at 30°C to 50°C.
to give a compound of formula I 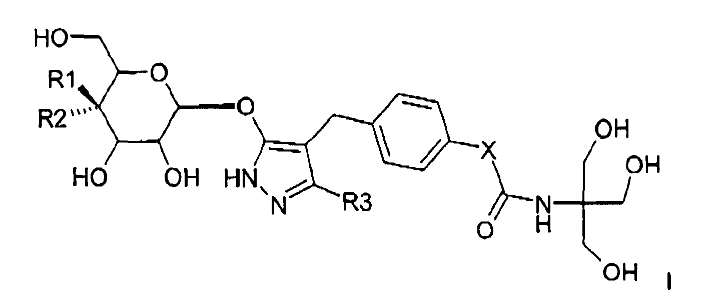
in which X, R1, R2 and R3 are as defined above;
and subsequently the compound of the formula (I) is purified by conventional purification methods such as crystallization or chromatography, preferably by crystallization from a solvent or a mixture of a plurality of solvents such as alkanes, aromatic compounds, halogenated solvents, ethers, ketones, esters, alcohols or water, particularly preferably by crystallization from alcohols or alcohols/water mixtures, very particularly preferably by crystallization from methanol/water.

In a further preferred embodiment the compound of formula VII is 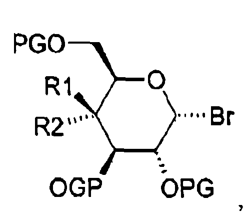 where
PG is benzoyl or acetyl;
R1 is H; and
R2 is F;
and the resulting products and intermediates of the process steps C and D are the β-glycosidic products: 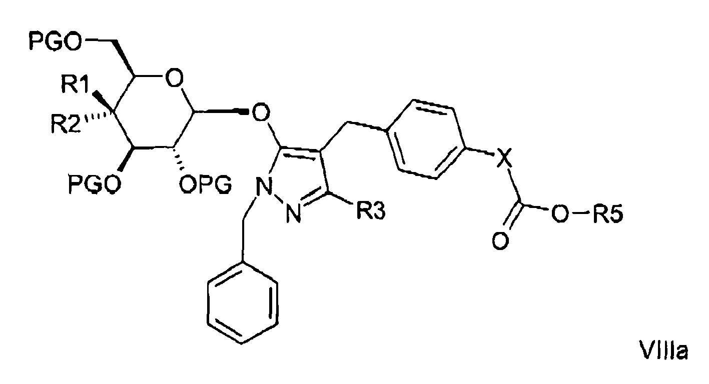
in which PG, X, R1, R2, R3 and R5 are as defined above;

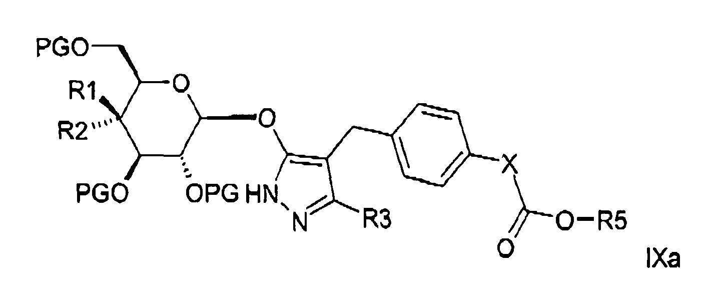

in which PG, X, R1, R2, R3 and R5 are as defined above;

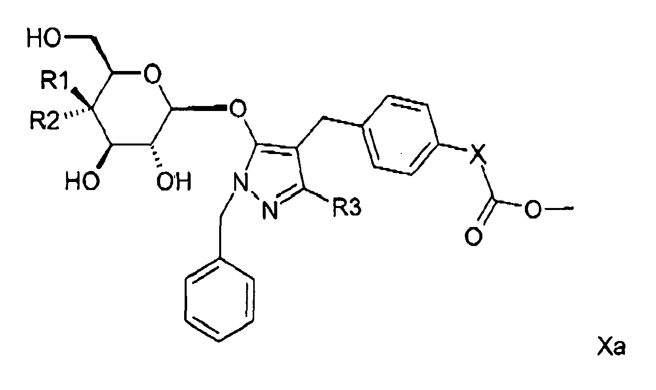

in which X, R1 and R3 are as defined above;

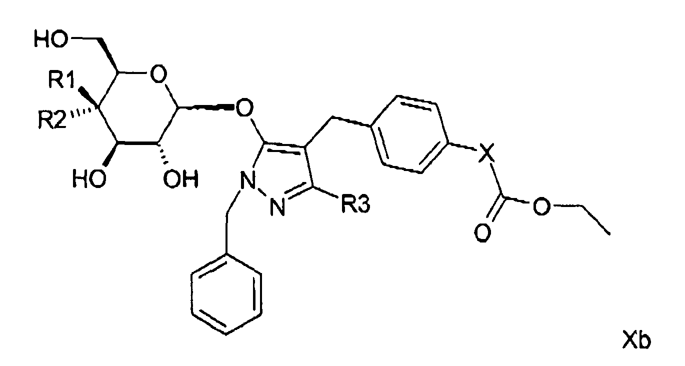

in which X, R1 and R3 are as defined above;

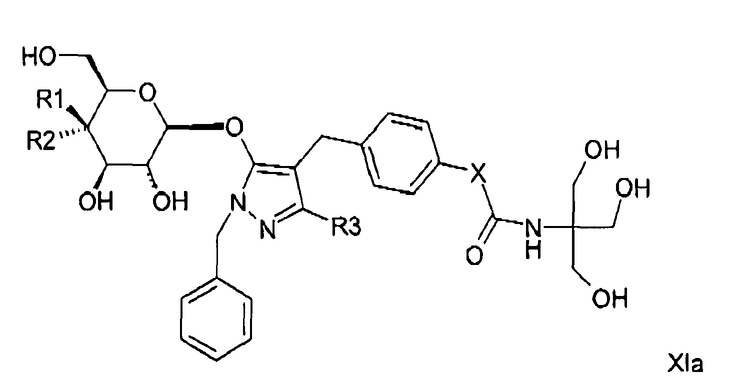

in which X, R1, R2 and R3 are as defined above;

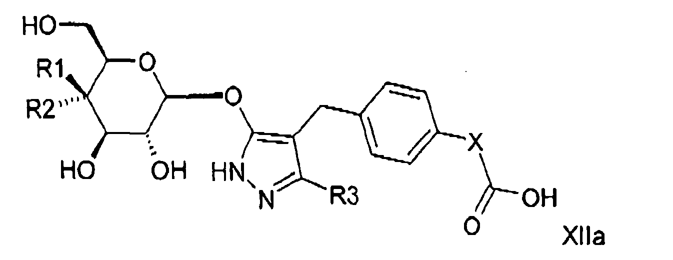

in which X, R1 and R3 are as defined above;

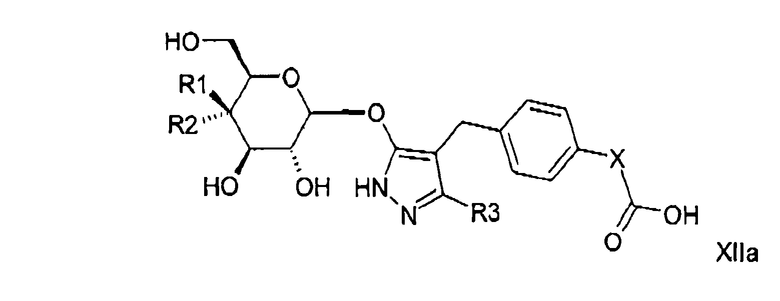

in which X, R1, R2and R3 are as defined above;

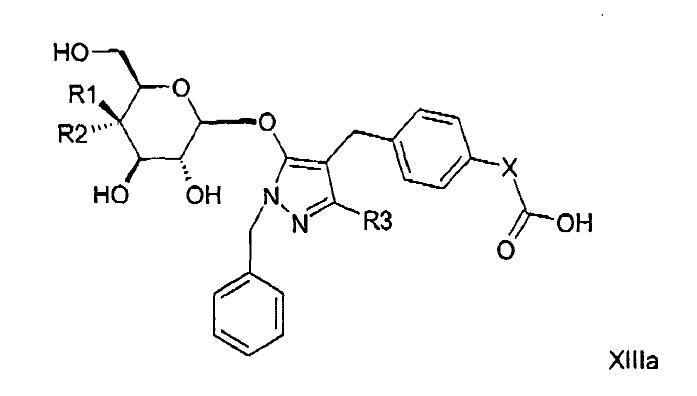

in which X, R1, R2 and R3 are as defined above;

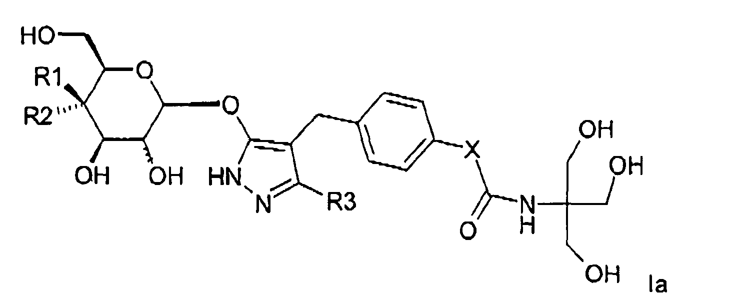

in which X, R1, R2 and R3 are as defined above.

Preference is given to a multistage process for preparing the compounds of the formula (I), in which
- R1: is H;
- R2: is F;
- R3: is isopropyl;
- R4, R5: are ethyl or methyl;
- PG: is benzoyl or acetyl;
- X: is(CH₂)₃.

The following examples illustrate the process without restricting it. Schemes 1 and 2 depict in an exemplary manner the preparation process.

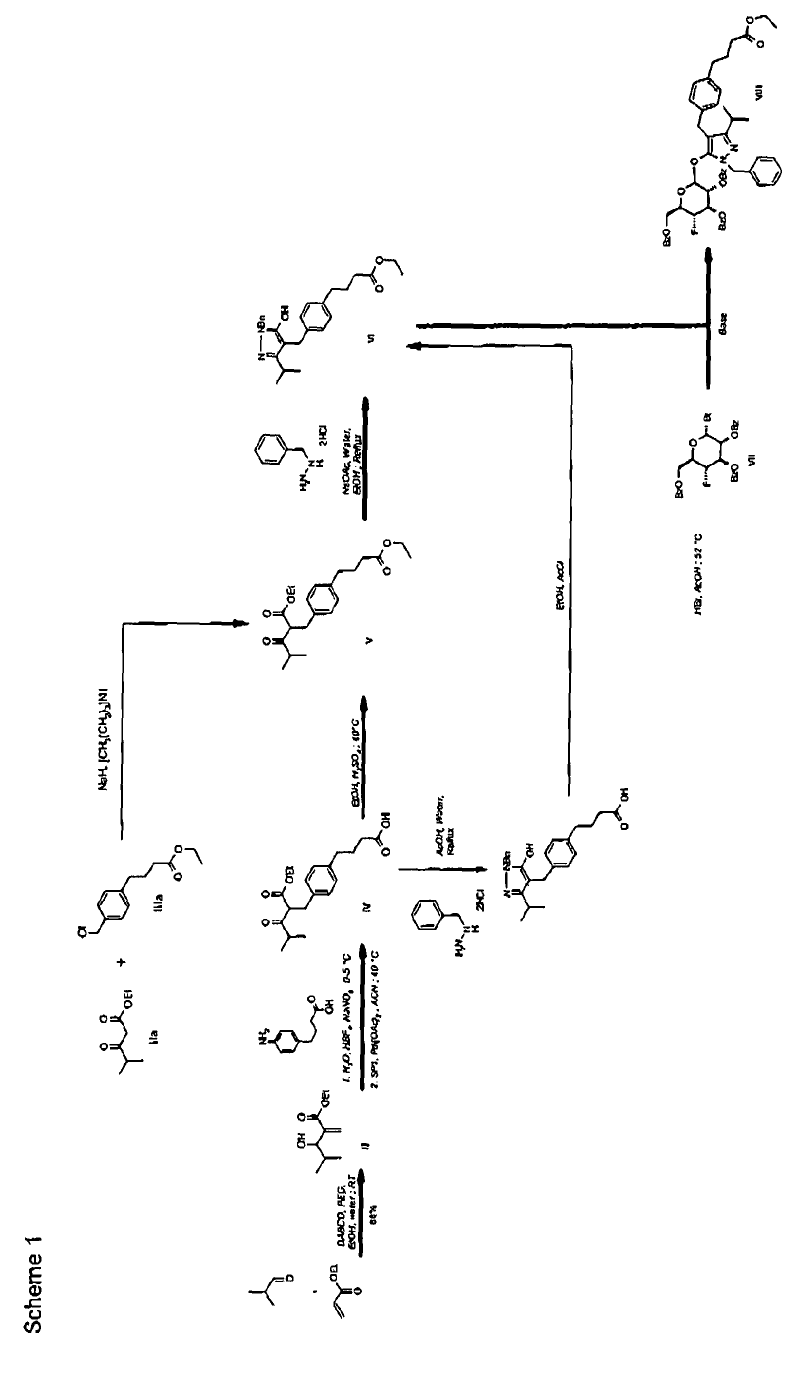

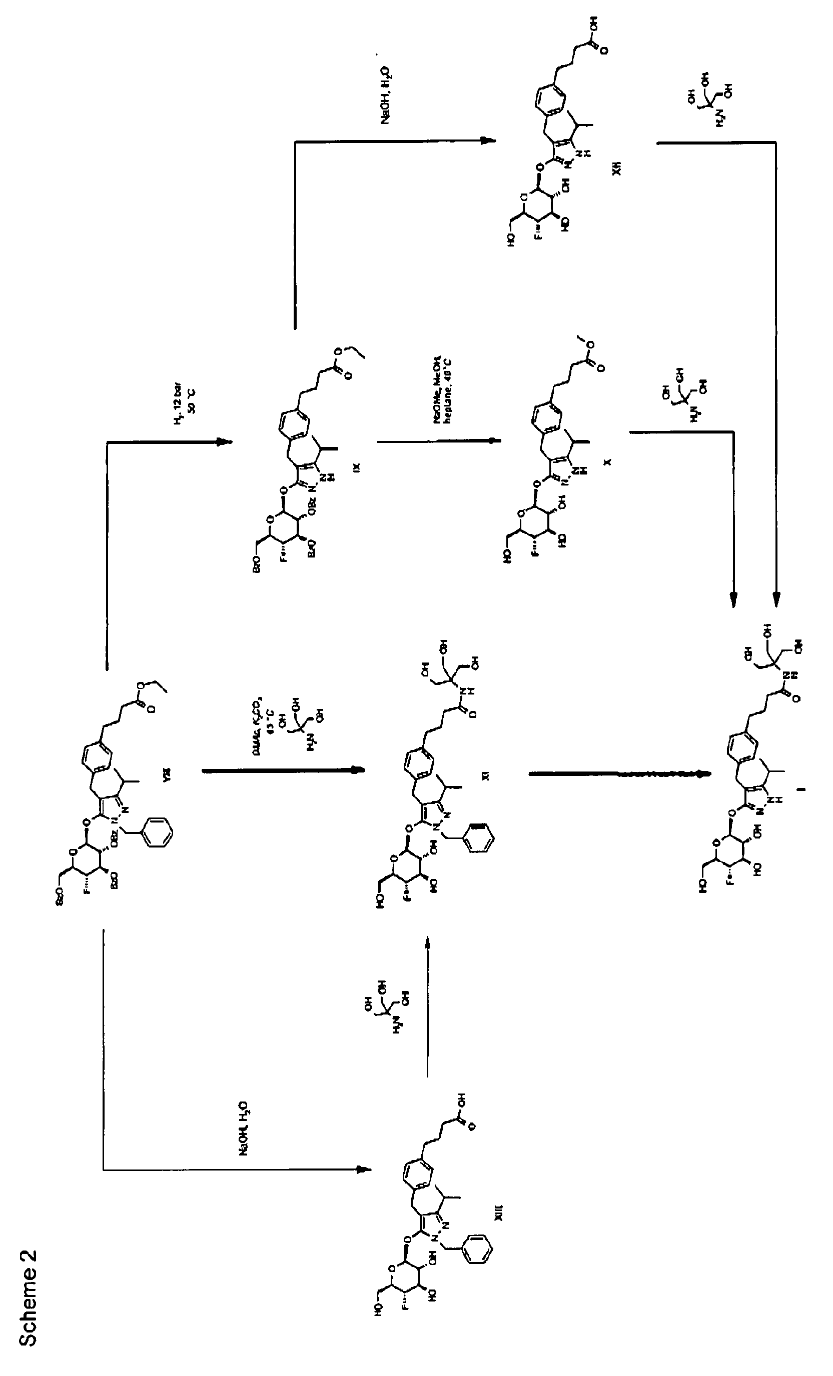

### Examples

### Example 1

### Compound II

### 3-Hydroxy-4-methyl-2-methylene-pentanoic acid ethyl ester

Ethylacrylate (230 ml, 2.16 mol, 1.0 eq), isobutyraldehyde (267 ml, 2.94 mol, 1.36 eq) and DABCO (167 g, 1.49 mol, 0.69 eq) were mixed together in the 3 component solvent mixture (300 ml PEG 400, 220 ml EtOH, 37 ml water) and stirred for 12 d. 1H-NMR (CDCl₃) showed about 20% of the acrylate remained in the reaction mixture. DABCO (77 g, 0.69 mol, 0.32 eq) and aldehyde (100 ml, 1.10 mol, 1.1 eq) were added and the mixture was allowed stirring for 7 d (NMR showed nearly complete consumption of the acrylate). Water (1 I) was added and the aqueous layer was extracted with MTBE (1x 800 ml, 3x 350 ml). The combined organic layers were washed with NaHSO₄ (2 M) until the pH of the resulting aqueous layer was about 3. The combined organic layers were washed with half conc. NH₄Cl (2x 150 ml), NaHCO₃ (300 ml), brine (400 ml), dried with MgSO₄ and concentrated to yield a yellow oil which was dried in vacuum (0.4 mbar) for 10 h. 335 g (90%) of the Baylis-Hillman adduct were obtained as a yellow oil. ¹H-NMR (500 MHz, d₆-DMSO): δ = 0.74 (d, *J* = 6.9 Hz, 3H), 0.88 (d, *J* = 6.8 Hz, 3H), 1.22 (t, *J* = 7.2 Hz, 3H), 1.68-1.82 (m, 1H), 4.09-4.18 (m, 2H), 4.18.4.23 (m, 1H), 4.87 (d, *J* = 5.2 Hz, 1H), 5.77-5.80 (m, 1H), 6.11-6.23 (m, 1H).

### Example 2

### Compound IV

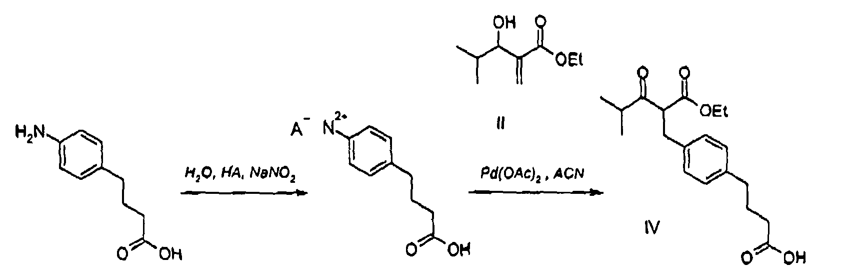

100 g 4-(4-Aminophenyl)-butyric acid in 400 ml water are treated with 43 ml conc. sulfuric acid 96-98% at 15-38°C (exothermic addition), followed by treatment with 38,5 g sodium nitrite in 100 ml water at -3°C to 0°C to form the corresponding diazonium salt. In a separate vessel 103 g compound II, 1.26 g palladium (II)-acetate and 5 g charcoal are suspended in 380 mL acetonitrile. The suspension is heated to 43°C and the cold diazonium solution is added at 42-49°C over 2 h and the reaction mixture is stirred at this temperature for another 2 h. After cooling to 25°C the resulting mixture is filtered over 25 g celite. The filter cake is washed with 530 ml ethyl acetate, followed by 50 ml water. After phase split the ethyl acetate phase is concentrated under reduced pressure and 200 g compound IV are obtained as a dark purple oily liquid, which is used in the next step without further purification.

### Example 3

### Compound V

200 g compound IV is solved in 650 ml ethanol and 4.7 ml sulfuric acid 96-98% are added. The solution is heated to 55°C and maintained at 55-58°C for 3 h to form the corresponding ethyl ester. The ethanol solution is cooled to ambient temperature and is used in the next step without treatment.

### Example 4

### Compound VI

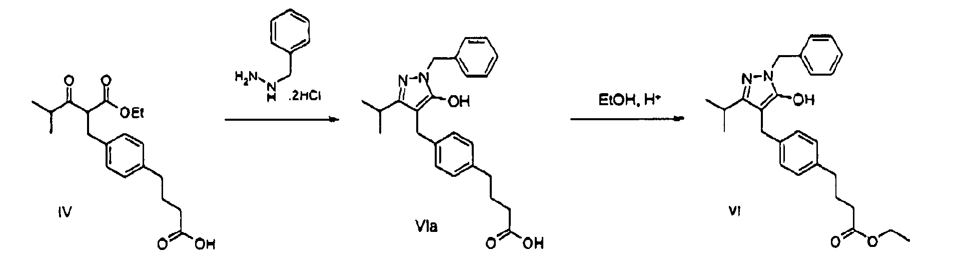

126 g sodium acetate and a solution of 162 g benzylhydrazine dihydrochloride in 250 ml water are added to the ethanol solution of compound V derived from example 3. The mixture is then heated to reflux (83°C) and maintained at this temperature for 6 h before cooling to ambient temperature. A first portion of 205 ml water are added over 15 minutes and the suspension is cooled over night at ambient temperature. The suspension is the cooled to 5°C and a second portion of 205 ml water is added. The slurry is stirred for another 1.5 h at 5°C, filtered, washed once with 200 ml ethanol/water (3:2) and four times with 100 ml water. The wet filter cake is then slurried for 2 h in 750 ml *tert.*-butyl methyl ether, filtered, washed with 150 ml *tert.-*butyl methyl ether and finally dried under reduced pressure to afford 156.9 g compound VI as a grey crystalline powder (65% yield over three steps based on 4-(4-Aminophenyl)-butyric acid).

### Example 5

### Compound IV

### 2-[4-(3-Carboxy-propyl)-benzyl]-4-methyl-3-oxo-pentanoic acid ethyl ester

Aniline (15 g, 84 mmol) was ground in a mortar and then placed in a 3-neeked flask. Water (30 ml), followed by 50 ml aq. HBF₄ (50%) were added and the mixture was cooled to -3°C (inner temperature). Sodium nitrite (6.01 g, 87 mmol), in 20 ml water, was added so slowly that the inner temp. was always below 0.5°C. After complete addition of the nitrite, the Baylis-Hillman adduct (compound II) (20.4 g, 119 mmol), in 200 ml acetonitrile, was added, followed by 5% Palladium acetate (inner temp 6°C). The mixture was then heated up to 65°C for 90 min. The heating was removed and the organic solvent was removed. The brown residue was brought to pH 9.5 with 2 M NaOH. The aqueous layer was extracted with MTBE (3x 100 ml). The combined organic layers were washed with water 60 ml (combined with the other basic aqueous layer), 100 ml brine (rejected), dried with MgSO4 and concentrated to yield excess Baylis-Hlllman adduct as a yellow oil. The basic aqueous layers were brought to pH 3.5 with NaHSO₄ (solid). The aqueous layer was extracted with AcOEt (1x 150 ml, 2x 50 ml). The combined organic layers were dried with MgSO₄ and concentrated to yield a brown oil, which was purified by chromatography on silica (n-heptane/AcOEt 4:1->3:1->2:1) to yield the product (17.6 g, 52.6 mmol, 63%) as a yellow oil. ¹H-NMR (500 MHz, d₆-DMSO): δ = 0.79 (d, J = 6.8 Hz, 3H), 0.96 (d, *J* = 6.8 Hz, 3H), 1.09 (t, *J* = 7.1 Hz, 3H), 1.71-1.81 (m, 2H), 4.00-4,08 (m, 2H), 4,16 (dd, *J* = 7.6, 7.8 Hz, 1H), 7.05-7.13 (m, 4H), 12.03 (bs, 1H); HPLC: t_{R} = 1.46 min (YMC J' sphere ODS H 80 24x2.1mm, 4µm, A: H₂O+0.05% TFA, B: MeCN, 4%→ 95% B in 2 min, 1mL/min, 30°C); Mass (ES+) (C₁₉H₂₆O₅): calcd. 334, found 335 [M+H]⁺.

### Example 6

### Compound Vla

### 4-[4-(1-Benzy)-5-hydroxy-3-isopropy)-1H-pyrazo)-4-ylmethyl)-phenyl]-butyric acid

Benzyl hydrazine (10.5 g, 53.8 mmol, 1.8 eq.) was suspended in 15 ml H₂O. NaOH (4.42 g, 111 mmol, 3.7 eq) was added at 0°C. The pH was adjusted from 11 to 6 by addition of AcOH. Then 10.0 g (29.9 mmol, 1 eq) β-keto ester (compound V), dissolved in 45 ml AcOH, were added and the mixture was refluxed for 4 h. After cooling to room temperature water (30 ml) and AcOEt (80 ml) were added. The phases were separated and the aqueous layer was extracted with AcOEt (3x 50 ml). The combined organic layers were washed twice with 20 ml sat aqueous NH₄Cl, dried with Na₂SO₄ and concentrated to yield a brown-orange oil. The oil was dried by azeotropic distillation with toluene (2x 30 ml). An aliquot (1 g) of the crude product was taken and purified by column chromatography (AcOEt-> AcOEt/EtOH 20:1) to yield a white solid after crystallization from AcOEt. The seed crystals so obtained were added to the crude oil in AcOEt (30 ml). The formed crystals were filtered and dried on air to yield 4.8 g (41 %) of the pyrazolone as a red-brown solid.

HPLC: t_{R} = 1.18 min (YMC J' sphere ODS H 80 20x2.1mm, 4µm, A: H₂O+0.05% TFA, B: MeCN, 4%→ 95% B in 2 min, 1mL/min, 30°C); Mass (ES+) (C₂₄H₂₈N₂O₃): calcd. 392, found 393 [M+H]⁺.

### Example 7

### Compound VI

### 4-[4-(-Benzyl-5-hydroxy-3-isopropyl-1H-pyrazol-4-ylmethyl)-phenyl]-butyric acid ethyl ester

1.1 ml (15 mmol, 2 eq.) acetyl chloride was carefully added to 40 ml EtOH with water bath cooling. After 10 min 3.0 g (7.6 mmol) benzyl pyrazolone were added and the mixture was stirred for 4 h at rt. The solvent was removed, the residue oil was distilled with 3x 20 ml AcOEt and dried in vacuum to yield 3.4 g (>100%) of the ester as a yellowish viscous oil.

HPLC: t_{R} = 1.42 min (YMC J' sphere ODS H 80 20x2.1mm, 4µm, A: H₂O+0.05% TFA, B: MeCN, 4%→ 95% B in 2 min, 1mL/min, 30°C); Mass (ES+) (C₂₆H₃₂N₂O₃): calcd. 420, found 421 [M+H]⁺.

### Example 8

### Compound VIII

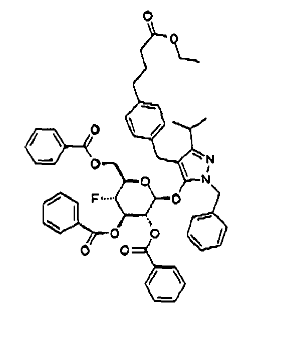

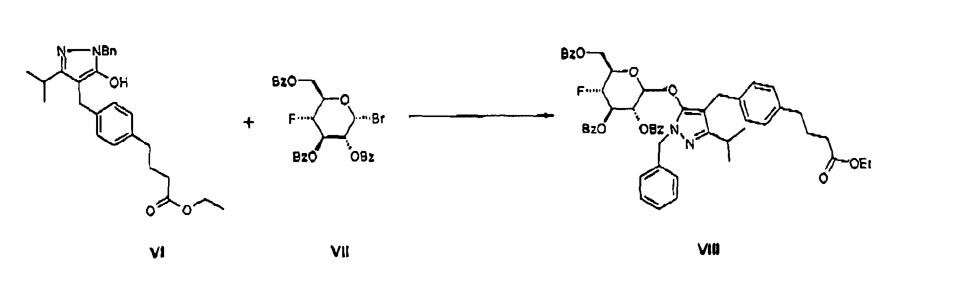

41.0 g (97.5 mmol. 1.2 eq) pyrazole VI was dissolved in 225 mL MeTHF at 50°C. To this solution 8.3 g (102 mmol, 1.25 eq) LiOtBu was added and the mixture was heated to reflux temperature. 45.3 g (81.2 mmol) flouro sugar VII was dissolved in 225 mL MeTHF and added dropwise over a period of 50 min to the refluxing reaction mixture. The reaction was kept at this temperature for 5 h.

The pH of the reaction mixture was adjusted to 7 using 5 mL 3M hydrochloric acid. After the addition of 70 mL water the aqueous phase was separated. The organic phase was extracted with 50 mL water and twice with 50 mL sodium chloride solution (4%). The solvent was exchanged to *n*-propanol by concentration and addition of 300 mL *n*-propanol followed by azeotropic distillation of another 70 mL.

During 2 h the solution was cooled to room temperature resulting in a slow precipitation of the product. During another 30 min the suspension was cooled to 0°C and was stirred for 1 h at this temperature. The product was filtered, washed four times with 60 mL ethanol and dried at 50°C under reduced pressure. 54 g (71%) of VIII was obtained.
HR-MS (ESI-FT-ICR): found [M+H]⁺: m/z=897.37698 (calculated for C53H54FN2010 897.37571)

### Example 9

### Compound VIII

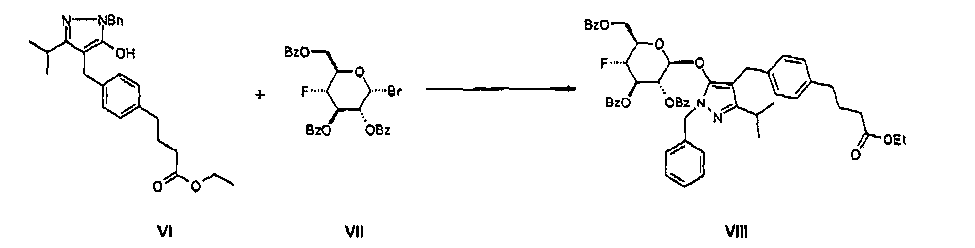

150 g (349 mmol) pyrazole VI and 197 g (349 mmol) flouro sugar VII were dissolved in 2720 mL acetonitrile and the mixture was heated to reflux temperature. 47 mL (334 mmol, 0.96 eq) triethylamine in 366 mL acetonitrile was added at this temperature over a period of 3 h. After the addition the solution was stirred for another 3h at reflux temperature. 800 mL water was added while keeping the temperature above 60°C. The mixture was cooled slowly to room temperature over night, The resulting suspension was cooled to 5°C and stirred at this temperature for 2.5 h. The product was filtered, washed twice with 440 mL ethanol, twice with 470 mL water and dried at 40°C under reduced pressure. 242 g (76%) of VIII was obtained.

### Example 10

### Compound XI

### 4-{4-[1-Benzyl-5-((2S,3R,4R,5S,6R)-5-fluoro-3,4-dihydroxy-6-hydroxymethyl-tetrahydro-pyran-2-yloxy)-3-isopropyl-1H-pyrazol-4-ylmethyl]-phenyl}-N-(2-hydroxy-1,1-bis-ydroxymethyl-ethyl)-butyramide

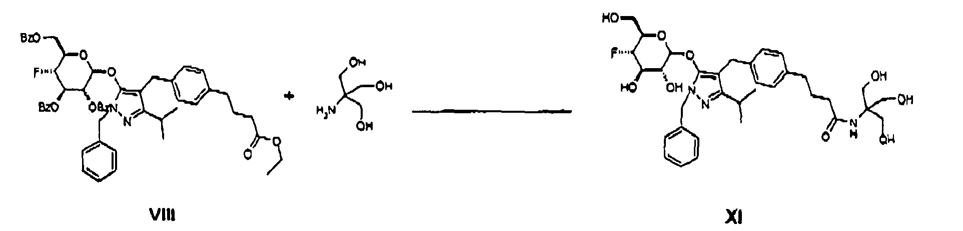

106 g (114 mmol) pyrazole VIII and 107.5 g (887 mmol, 7.8 eq) 2-amino-2-hydroxymethyl-propane-1,3-diol were dissolved in 900 mL *tert*-butanol. 26.5 g (228 mmol, 2.0 eq) potassium *tert*-butylate was added and the reaction mixture was heated to 45°C and stirred for 2.5 h at this temperature. 28.5 g concentrated sulphuric acid in 250 mL water and subsequently 800 mL water was added while keeping the temperature above 40°C. The organic solvent was distilled of under reduced pressure. 1200 mL ethyl acetate was added and the resulting biphasic mixture was stirred at room temperature over night. The organic layer was washed three times with 850 mL sodium hydrogen carbonate solution (5%) and twice with 850 mL sodium chloride solution (5%) and filtered though charcoal. The charcoal was washed three times with 200 mL ethyl acetate. The resulting solution of XI was concentrated to a volume of 150 mL and was used for the next step without further purification.

### Example 11

### Compound I

### 4-{4-[5-((2S,3R,4R,5S,6R)-5-Fluoro-3,4-dihydroxy-6-hydroxymethyl-tetrahydro-pyran-2-yloxy)-3-isopropyl-1H-pyrazol-4-ylmethyl]-phenyl}-N-(2-hydroxy-1,1-bis-ydroxymethyl-ethyl)-butyramide

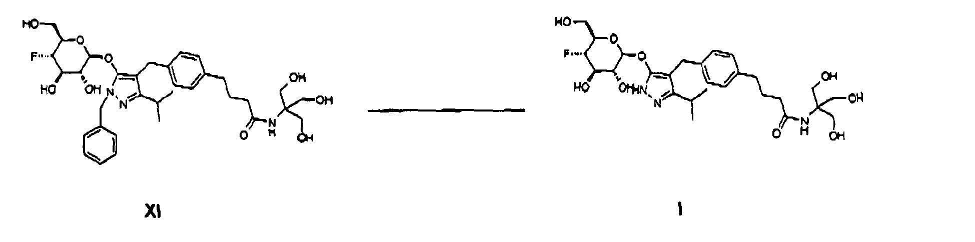

To a solution of pyrazole XI in ethyl acetate (total volume 150 mL) 650 mL methanol was added. 7.5 g palladium hydroxide on charcoal (20%, 55% water) was added to the solution. The reaction mixture was stirred for 21 h under a hydrogen atmosphere of 1 bar at 25°C. The catalyst was filtered of and washed with 525 mL water. The organic solvents were removed under reduced pressure and 165 mL ethyl acetate was added. The aqueous product layer was washed with 165 mL ethyl acetate and was filtered. The residual organic solvent was removed under reduced pressure. The product solution was cooled to 27°C and a few seed crystals were added. The suspension was stirred at 20°C over night and the product was filtered, washed with 35 mL water and dried under reduced pressure at 25°C. 34.2 g (52%, two steps) of I was obtained.

### Example 12

### Compound XI

### 4-{4-[1-B enzyl-5-((2S,3R,4R,5S,6R)-5-fluoro-3,4-dihydroxy-6-hydroxymethyl-tetrahydro-pyran-2-yloxy)-3-isopropyl-1H-pyrazol-4-ylmethyl]-phenyl}-N-(2-hydroxy-1,1-bis-ydroxymethyl-ethyl)-butyramide

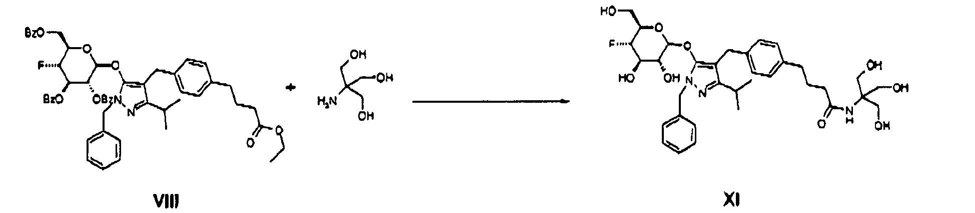

105 g (116 mmol) pyrazole VIII and 114 g (932 mmol, 8 eq) 2-amino-2-hydroxymethyl-propane-1,3-diol and 48.6 g potassium carbonate (348 mmol, 3 eq) were suspended in 400 ml N,N-dimethyl-acetamide. The reaction mixture was heated to 45°C and stirred for 7.5 h at this temperature. 750 ml 2-methyl-tetrahydrofurane was added and the mixture was cooled to 5°C. The suspension was filtered over charcoal and 250 ml 2-methyl-tetrahydrofurane was added. The filtrate was added to a solution of 200 g sodium chloride in 800 ml water and the phases were separated, The organic phase was washed twice with 5% NaCl solution and finally concentrated under reduced pressure. The residue was dissolved In 800 ml ethyl acetate and the solution was washed three times with 750 ml 5% NaCI solution. The organic phase was finally filtered over charcoal and concentrated to 150 ml under reduced pressure and was used for the next step without further purification.

### Example 13

### Compound IX

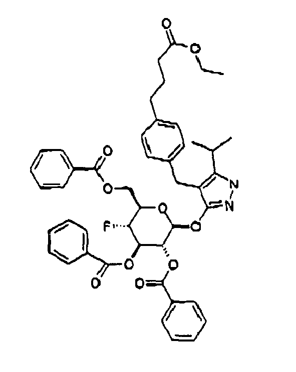

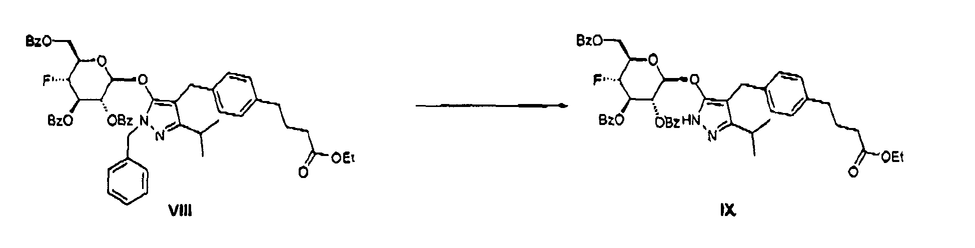

60 g (63 mmol) pyrazole VIII was dissolved in 210 mL THF and 3 g palladium hydroxide on charcoal (20%, 55% water) was added to the solution. 420 mL ethanol was added and the reaction mixture was stirred for 6 h under a hydrogen atmosphere of 6 bar at 70°C, The catalyst was filtered of and washed with ethanol. The solvents were removed under reduced pressure. The resulting crude product IX (55.6 g) was used for the next step without further purification.
HR-MS (ESI-FT-ICR): found [M+H]⁺: m/z=807.3308 (calculated for C46H48FN2O10 807.328756)

### Example 14

### Compound X

### 4-(4-[5-((2S,3R,4R,5S,6R)-5-Fluoro-3,4-dihydroxy-6-hydroxymethyl-tetrahydro-pyran-2-yloxy)-3-isopropyl-1H-pyrazol-4-ylmethyl]-phenyl}-butyric acid methyl ester

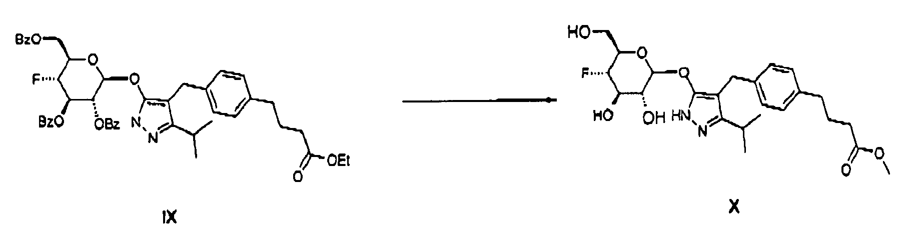

55.6 g pyrazole IX was dissolved in 600 mL methanol and 100 mL methanol was distilled to remove residual water. 3.9 g sodium methylate (25% solution in methanol) was added and the reaction mixture was stirred for 1 h at 35°C. The reaction mixture was adjusted to an apparent pH 5 using 0.67 mL concentrated sulphuric acid and concentrated to a volume of 120 mL. The resulting solution was washed four times with 500 mL *n*-heptan and concentrated under reduced pressure. The resulting crude product X (30 g) was used for the next step without further purification.

### Example 15

### Compound I

### 4-{4-[5-((2S,3R,4R,5S,6R)-5-Fluoro-3,4-dihydroxy-6-hydroxymethyl-tetrahydro-pyran-2-yloxy)-3-isopropyl-1H-pyrazol-4-ylmethyl]-phenyl}-N-(2-hydroxy-1,1-bis-ydroxymethyl-ethyl)-butyramide

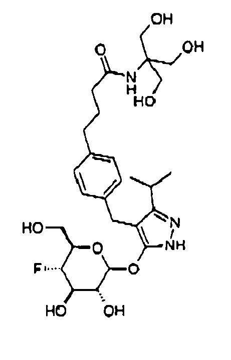

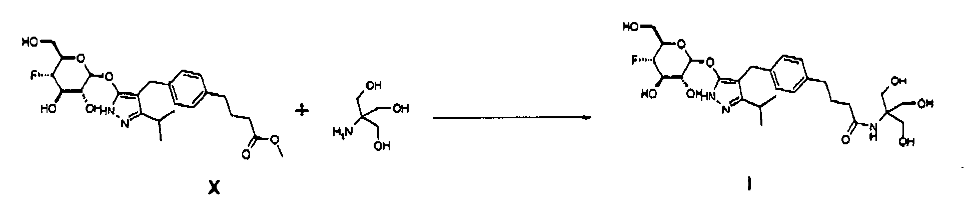

27.2 g pyrazole X was dissolved in 38 mL methanol. This solution was added over a period of 20 min to a solution of 164 g (1.3 mol, 25 eq) 2-amino-2-hydroxymethyl-propane-1,3-diol in 110 mL water. The reaction mixture was stirred for 5 h at 50°C.

After cooling the mixture to room temperature excess of 2-amino-2-hydroxymethyl-propane-1,3-diol was filtered of. After concentration of the filtrate it was filtered again, The resulting solution of I was concentrated to a mass of 142 g. The product was purified by chromatography using Sepabeads SP70 and water and ethanol as eluent and by treatment with ion exchanger. After lyophilisation 21.3 g (68%, 3 steps) I was obtained.
MS (TOF MS ES+): found [M+H]⁺: m/z=570.28 (calculated for C27H41 FN309 570.28)

### Example 16

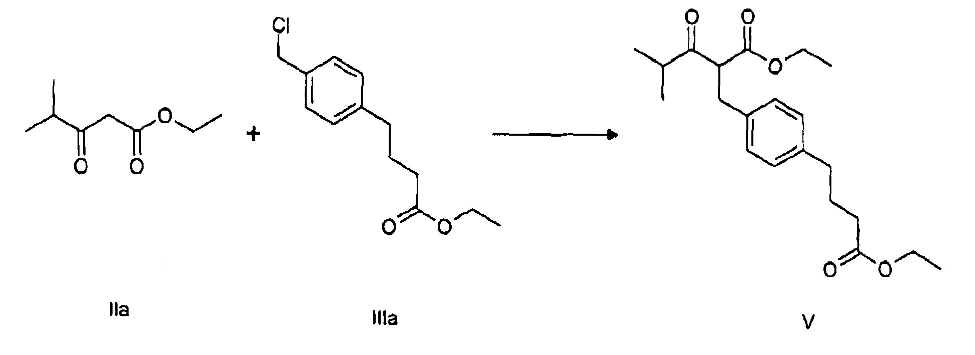

### Compound V

### 2-[4-(3-Ethoxycarbonyl-propyl)benzyl]-4-methyl-3-oxo-pentanoic acid ethyl ester

3.62 g (60%, 91 mmol, 1.15 eq) sodium hydride was suspended in 200 mL tetrahydrofurane. 19.7 g (118 mmol, 1.5 eq) ethylisobutyryl acetate was added dropwise within 8 min at 20 °C. A vigorous gas formation was observed. 2.9 g (7.9 mmol, 0.1 eq) tetrabutylammoniumiodid and 20.0 g (79 mmol, 1.0 eq) 4-(4-chloromethyl-phenyl)-butyricacid ethylester were added. The reaction mixture was stirred for 3 h at 60 °C. After complete conversion the mixture was cooled to room temperature and the suspension was neutralised (pH 7) with 13 mL 0.5 M HCl. 45 mL water was added and the layers were separated. The organic layer was diluted with 70 mL ethyl acetate and washed five times with 100 mL sodium chloride solution (4%). The solvents were removed under reduced pressure. The resulting yellow oil contained about 7% of ethylisobutyryl acetate. This impurity was removed by stirring the crude product at 70 °C under vacuum (0.02 mbar). 29.3 g 8 (94%, 92% purity) was obtained.

### Example 17

### Compound I

### 4-{4-[3-((2S,3R,4R,5S,6R)-5-Fluoro-3,4-dihydroxy-6-hydroxymethyl-tetrahydro-pyran-2-yloxy)-5-isopropyl-1 H-pyrazol-4-ylmethyl]-phenyl}-N-(2-h ydroxy-1,1-bis-hydroxymethyl-ethyl)

To a solution of 86 g (107 mmol) Compound IX in 950 mL ethanol 624 mL sodium hydroxide solution (1 M, 623 mmol, 5.8 eq) was added dropwise. The mixture was stirred for 6 h at room temperature and neutralised (pH 9) with 100 mL 1 M HCl solution. The solution was concentrated to a volume of 650 mL to remove ethanol. 320 mL 1 M HCl solution, 100 mL water and 800 mL diisopropylether were added. The product was isolated by filtration, was washed with water and diisopropylether and dried in vacuum at 40 °C. The crude product (48,4 g) was suspended in 500 mL diisopropylether stirred for 1 h, isolated by filtration, washed with diisopropylether and dried in vacuum at 40 °C. 47.1 g (92%) XII was obtained as slightly yellow powder.

### Compound I

### 4-{4-[5-((2S,3R,4R,5S,6R)-5-Fluoro-3,4-dihydroxy-6-hydroxymethyl-tetrahydro-pyran-2-yloxy)-3-isopropyl-1H-pyrazol-4-ylmethyl]-phenyl}-N-(2-hydroxy-1,1-bis-ydroxymethyl-ethyl)-butyramide

1 g (2.1 mmol) XII and 300 mg (3.2 mmol, 1.5 eq) tris were dissolved in 3 mL NMP. 734 mg (3.0 mmol, 1.4 eq) EEDQ (2-ethoxy-1-ethoxycarbonyl-1,2-dihydrochinolin) was added and the reaction mixture was stirred at 55°C for 16h. After 6h 91 mg tris and after 8h 100 mg EEDQ were added to complete the reaction. In order to deplete impurities the mixture was treated portion wise with 220 mL 1 M LiOH solution and stirred at room temperature for 45h. 15 mL water was added and the aqueous layer was extracted seven times with 40 mL DCM and twice with 20 mL propyl acetate to remove NMP and impurities. The aqueous product solution was neutralised (pH 8) with 100 µL 1 M LiOH solution. The solution was concentrated from 25 g to 9 g and few seed crystals were added. The mixture was stirred for 4h at 0°C and the product
was isolated by filtration washed twice with 3 mL of cold water and dried in vacuum at 40 °C. 0.73 g (57%) of compound I was obtained as colourless solids.

### Example 18

### Compound X

40 g (45 mmol) of compound VIII was suspended in 200 mL methanol and stirred at 40 °C for 3 h. 1.8 mL (9.5 mmol, 0.2 eq) sodium methylate was added. 45 mL THF were added and resulted in a better stirrable and a clear solution after 10 min. The reaction mixture was stirred for 5 h at 40 °C.

A quarter of the solution was trated with 66 µl concentrated sulphuric acid (pH 3) and neutralised (pH 5-6) with 0.5 mL saturated NaHCO₃ solution. The solution was concentrated under reduced pressure and three times 20 mL water was added and the mixture was concentrated each time under reduced pressure. The residue was extracted with ethyl acetate and water. The organic layer was concentrated and suspended twice in 60 mL DIPE and filtered each time. 4.2 g (64%) compound X was isolated as slightly yellow solids.

### Compound XI

4.0 g (6.5 mmol) SP8 methyl ester was dissolved in 16 mL NMP. 1.7 g tris (14 mmol, 2.2 eq) and 0.17 g (2,0 mmol, 0.3 eq) LiOtBu were added. The mixture was stirred at room temperature for 18 h. The reaction mixture was added to 65 mL water and 10 mL NaHCO₃ solution (8%) and extracted with 40 mL MeTHF. The aqueous layer was extracted with 40 mL MeTHF and the combined organic layers were washed four times with 20 mL NaCl solution (10%) and were concentrated under reduced pressure to give 3.9 g (88%) XI as slightly yellow solid.

### Example 19

### Compound XIII

6.0 g (10.5 mmol) VIII was dissolved in 40 mL THF, 20 mL water and 10 mL t-butanol. 10.5 mL (1M, 10.5 mmol, 1.0 eq) NaOH solution was added in 3 portions within 3 h. The first 2 h the reaction temperature was kept at 40 °C and stirred afterwards at room temperature over night. The reaction mixture was concentrated to a volume of 20 mL, diluted with 30 mL water and extracted with 100 mL DIPE. Traces of DIPE in the aqueous layer were removed by azeotropic distillation. The product solution was cooled to 0 °C and 9.5 mL 1M HCl solution was added (pH 5). The aqueous product was isolated by filtration, washed with water and dried in vacuum. 5.6 g (94%) XIII was isolated as slightly brown solids.

### Compound XI

90 mg (0.162 mmol) XIII was dissolved in 1 mL DMF and the solution was cooled to 0 °C. 42 mg (0,37 mmol, 2.3 eq) N-ethylmorpholine and 22 mg (0.161 mmol, 0.99 eq) CASIBUT were added and the mixture was stirred for 5 min at 0 °C. 20 mg (1.65 mmol, 1.02 eq) tris were added and the reaction mixture was stirred at 0 °C for 2 h. 84 area% of XI were present in the reaction mixture (identified by LC MS). 8 area% of XIII were still present, too.

## Claims

1. A process for preparing compounds of the general formula (I): 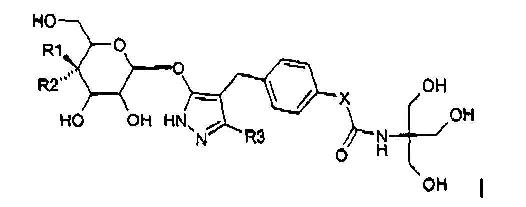 in which the meanings are
R1 H and R2 F; or
R1 F and R2 H; or
R1 F and R2 F;
R3 (C₁-C₈)-alkyl, where one, more than one or all hydrogen(s) may be replaced by fluorine; .
X (C₁-C₃)-alkylene, (C₂-C₃)-alkenylene;
which comprises applying a multistage process in which
A. Preparation of the beta-keto-ester
A.1. the component of the formula (II) 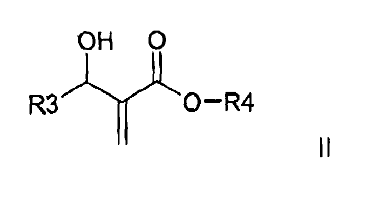 in which
R3 is (C₁-C₈)-alkyl, where one, more than one or all hydrogen(s) may be replaced by fluorine;
R4 is (C₁-C₈)-alkyl;
is reacted with 0.5 to 2 equivalents of compound of the formula (III) 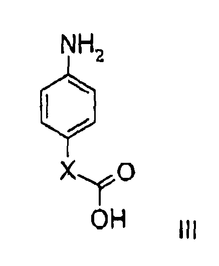 in which
X is (C₁-C₃)-alkylene, (C₂-C₃)-alkenylene;
by first treating compound of formula III in the presence of from 0.1 to 10 equivalents, of one or more acids in a suitable solvent, at from -50°C to 0°C, with 1.0 to 1.5 equivalents of NaNO₂, and
by adding this mixture to a mixture of 0.8 to 1.5 equivalents of the component of formula II comprising a catalyst, in a suitable solvent, which is watermiscible, at 0°C to 100°C;
to give a compound of the formula (IV), 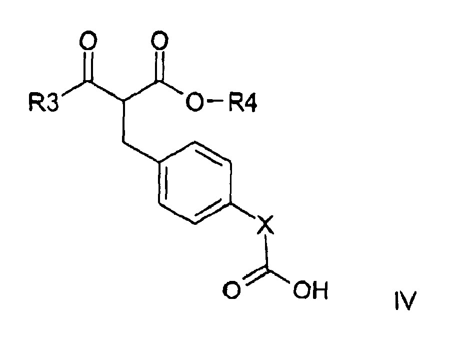
in which X, R3 and R4 are as defined above;
and
optionally the compound of the formula (IV) is purified by conventional purification methods such as crystallization, distillation or chromatography;
B. Preparation of pyrazolones
B.1. A compound of formula IV 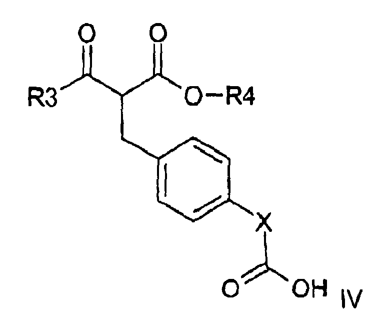
in which X, R3 and R4 are as defined above;
is converted to a compound of formula V 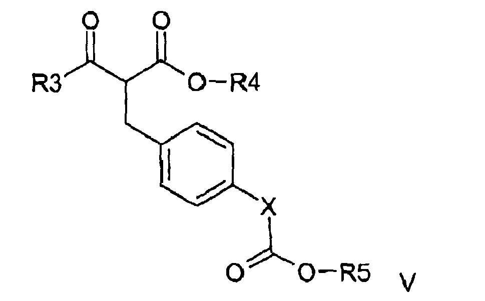
where R5 is is (C₁-C₆)-alkyl and X, R3 and R4 are as defined above;
by treatment with R5-OH in the presence of an acidic catalyst and a water removing reagent;
and the compound of formula V is subsequent reacted with 0.8 to 1.5 equivalents of the compound 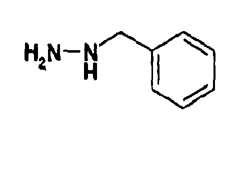 in the presence of an acidic catalyst at from -50°C to +150°C, to give a compound of the formula (VI) 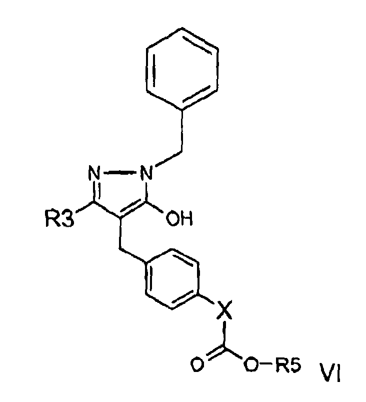
in which X, R3 and R5 are as defined above;
and
optionally the compound of the formula (VI) is purified by conventional purification methods such as crystallization, distillation or chromatography;
or
B.2. A compound of formula IV 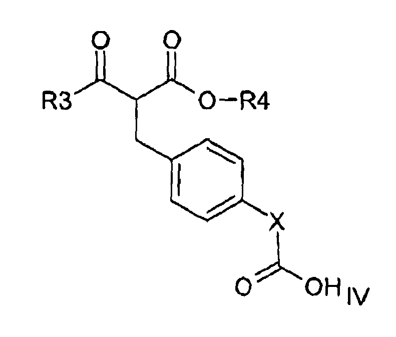
in which X, R3 and R4 are as defined above.
is reacted reacted with 0.8 to 1.5 equivalents of the compound 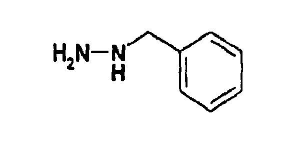 in the presence of an acidic catalyst at from -50°C to +150°C,
to give a compound of formula VIa 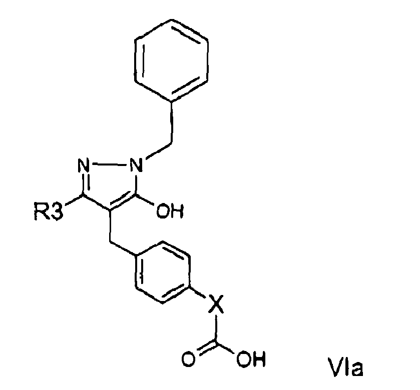
in which X and R3 are as defined above;
which is reacted further in the presence by treatment with R5-OH in the presence of an acidic catalyst and a water removing reagent;
to give a compound of formula VI 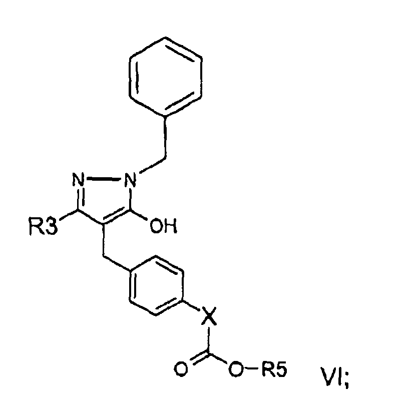 and
optionally the compound of the formula (VI) is purified by conventional purification methods such as crystallization, distillation or chromatography;
C. Preparation of pyrazolone glycoside
C.1. A compound of the formula (VI) 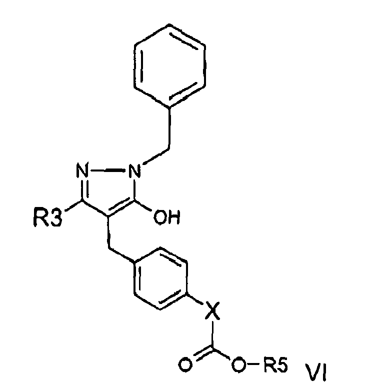
where R3 and R5 are defined as described above
is reacted with a sugar derivative of the formula (VII) 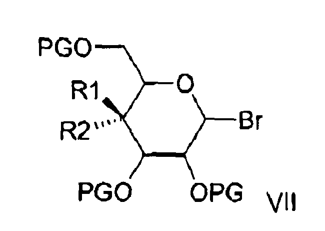 in which
R1 and R2 are defined as described above;
PG is an OH protective group;
by adding 0.95 to 1.2 equivalents of a Li base to compound VI In an ethereal solvent;
and the Li salt of the compound of the formula (VI) is subsequently reacted with 0.5 to 2 equivalents of the compound of the formula VII at 40°C to 120°C;
to give the compound of the formula (VIII); 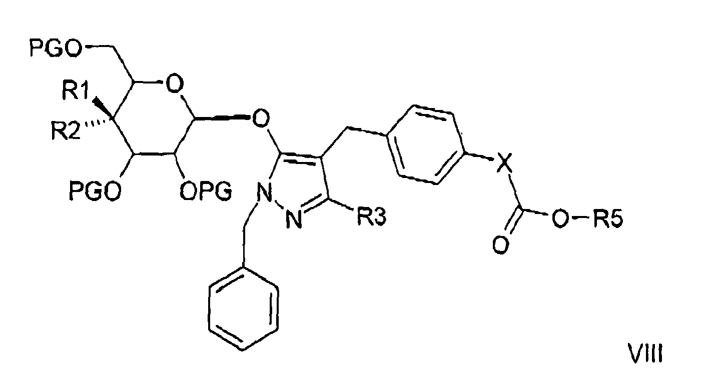
in which PG, X, R1, R2, R3 and R5 are as defined above;
and
optionally the compound of the formula (VIII) is purified by conventional purification methods such as crystallization, distillation or chromatography;
or
C.2. the compound of the formula (VI) 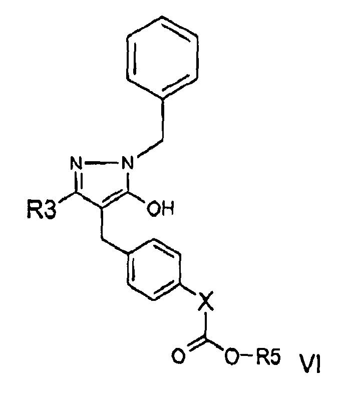 is reacted with 0.5 to 2 equivalents the compound of the formula VII, 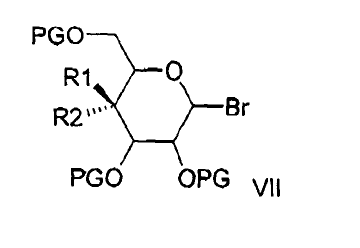 in which
R1 and R2 are defined as described above;
PG is an OH protective group;
in an inert solvent in the presence of a trialkyl amine base at 40°C to 120°C, to give a compound of formula VIII 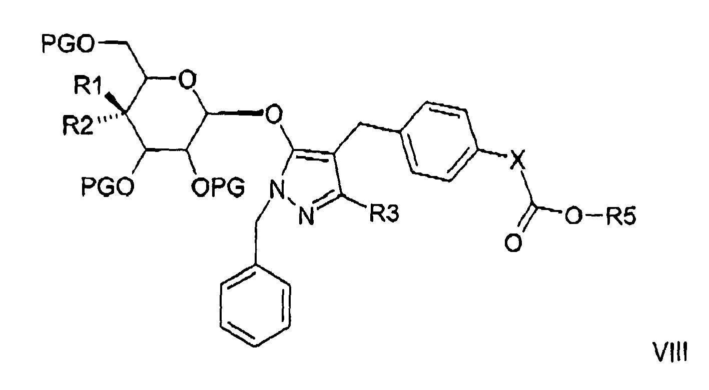
in which PG, X, R1, R2, R3 and R5 are as defined above;
and
optionally the compound of the formula (VIII) is purified by conventional purification. methods such as crystallization, distillation or chromatography;
D. Preparation of the pyrazole-glycoside derivative
D.1. The compound of formula VIII 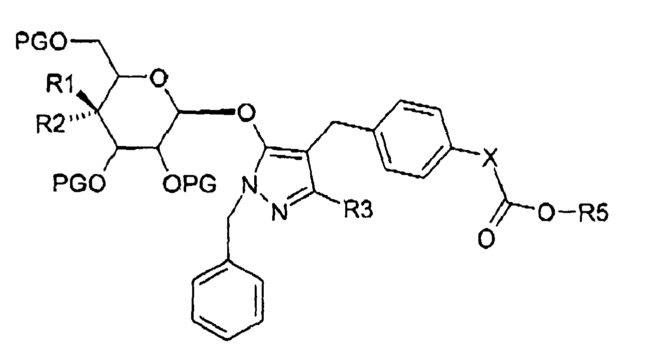
in which PG, X, R2 , R2, R3 and R5 are as defined above;
Is converted to compound IX 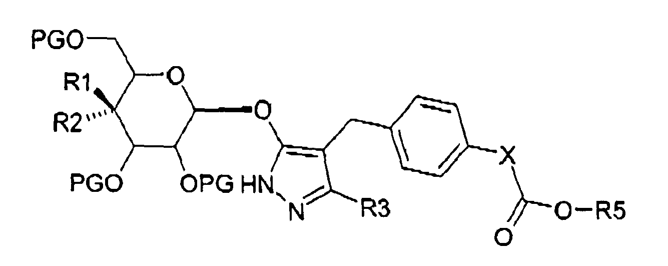
in which PG, X, R1, R2, R3 and R5 are as defined above;
by hydrogenolysis with a transition metal catalyst in a suitable solvent at +10°C to +80°C at a hydrogen pressure of 1 bar to 60 bars;
The resulting free N unprotected 3-pyrazolone can be isolated or used in crude, unisolated form;
and the compound of the formula IX is subsequent converted to a compound of formula X 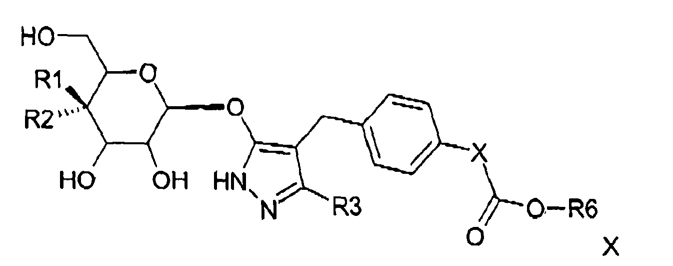
in which X, R1, R2 and R3 are as defined above;
R6 is (C₁-C₆)-alkyl;
by reaction with an excess of a salt of an alcohol In alcoholic solvent at +10°C to +94°C;
and subsequent reacting with an excess of a concentrated aqueous solution of tris(hydroxymethyl)aminomethane to an aqueous solution of the compound of formula X
gives the compound of formula I 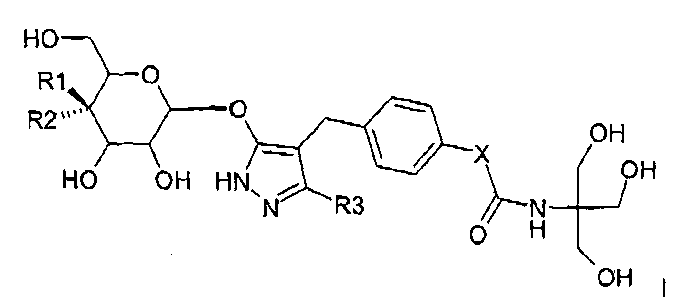
in which X, R2, R2 and R3 are as defined above;
and subsequent
isolation of the product is achieved by conventional methods like resin chromatography of the crude reaction mixture.
Or
D.2. a compound of formula VIII 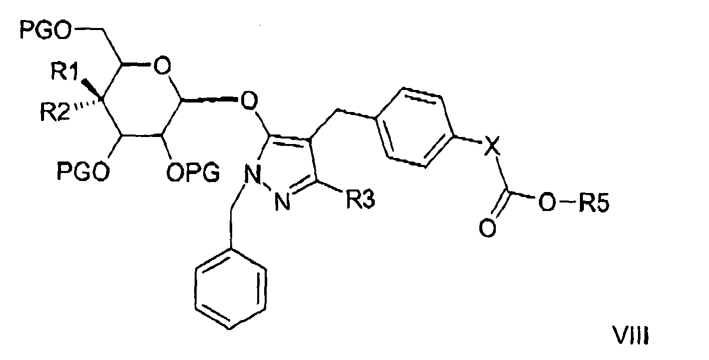
in which PG, X, R1, R2, R3 and R5 are as defined above;
is reacted with an excess tris(hydroxymethyl)aminomethane in an alcoholic solvent in the presence of an alcoxide
to give a compound of formula XI 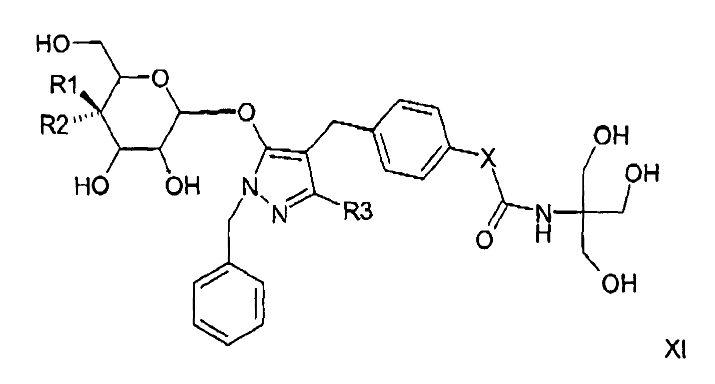
in which X, R1, R2 and R3 are as defined above;
and
subsequent hydrogenolysis with a heterogeneous platinum metal catalyst on a solid support at a hydrogen pressure of 1 bar to 60 bars at 20°C to 90°C
to give a compound of formula I 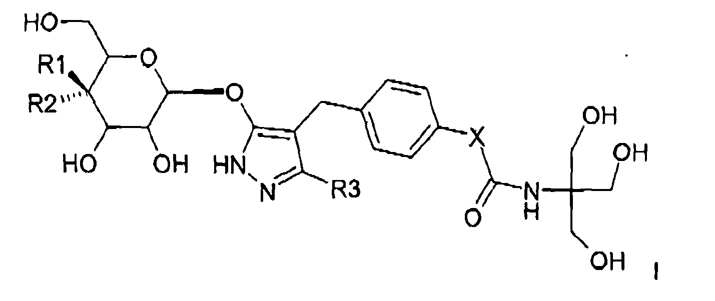
in which X, R1, R2 and R3 are as defined above;
and subsequently the compound of the formula (I) is purified by conventional purification methods such as crystallization or chromatography.

2. A process for preparing a compound of the general formula (I) as claimed in claim 1, where the process step A. has the following meaning
A.2. the component of the formula (Ila) 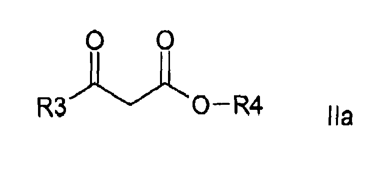 in which
R3 is (C₁-C₈)-alkyl, where one, more than one or all hydrogen(s) may be replaced by fluorine;
R4 is (C₁-C₈)-alkyl;
is reacted with 0.5 to 2 equivalents of compound of the formula (Illa) 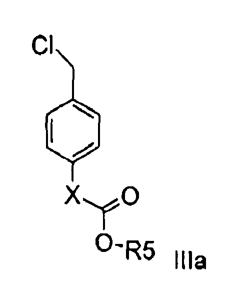 in which
X is (C₁-C₃)-alkylene, (C₂-C₃)-alkenylene;
R5 is (C₁-C₆)-alkyl;
in the presence of from 0.1 to 10 equivalents of a strong non-nucleophic base, optionally in the presence of a phase transfer catalyst, with 0.05 to 0.5 equivalents of a phase transfer catalyst, in a suitable solvent at from -50°C to 50°C to give a compound of the formula (V),

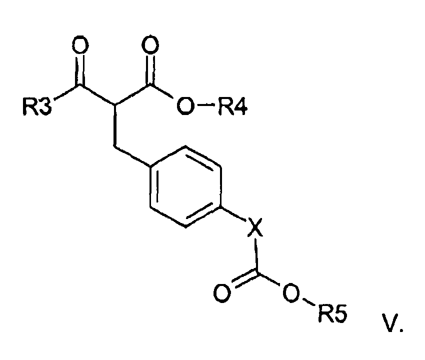

3. A process for preparing a compound of the general formula (I) as claimed in claim 1 or 2, where the process step D. has the following meaning
D. Preparation of the pyrazole-glycoside derivative
0.3 The compound of formula VIII 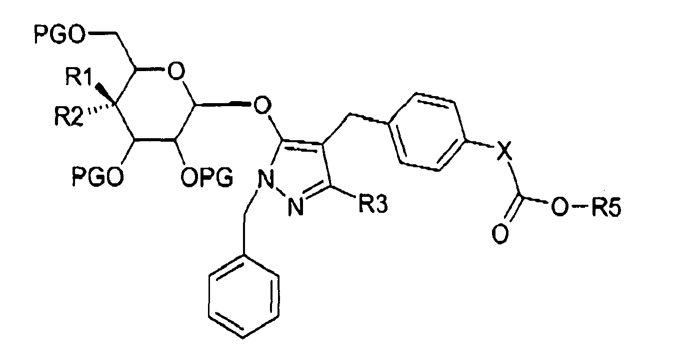
in which PG, X, R1, R2, R3 and R5 are as defined above;
is converted to compound IX 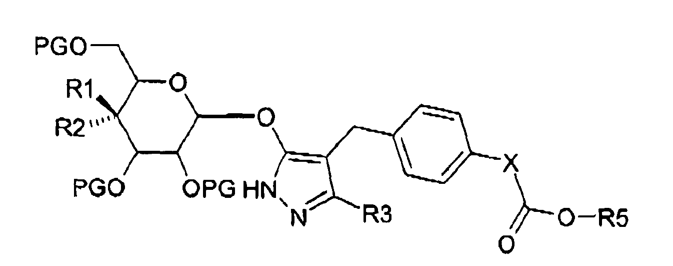
in which PG, X, R1, R2, R3 and R5 are as defined above;
by hydrogenolysis with a transition metal catalyst, in a suitable solvent at +10°C to +80°C at a hydrogen pressure of 1 bar to 60 bars;
and optionally the compound of formula IX can be isolated using techniques known to those skilled in the art, but it can also be used in crude, unisolated form;
and the compound of the formula IX is subsequent converted to a compound of formula XII 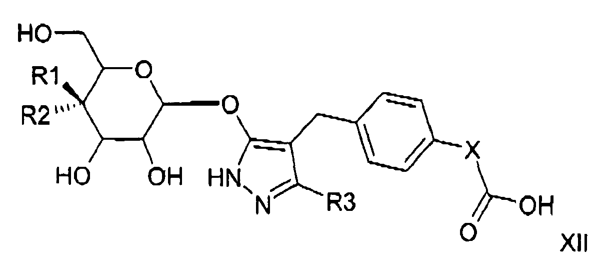
in which X, R1, R2 and R3 are as defined above;
by reaction with a base in an aqueous solvent at +10°C to +90°C;
and subsequent reacting with an excess of tris(hydroxymethyl)aminomethane in a suitable solvent with an amide forming reagent to an solution or suspension of the compound of formula XII
to give the compound of formula I 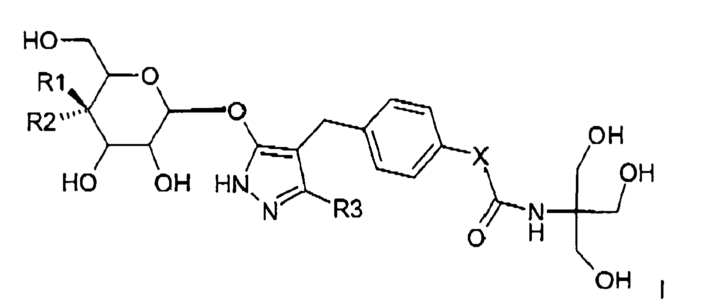
in which X, R1, R2 and R3 are as defined above;
and subsequent
isolation of the product is achieved by conventional methods like resin chromatography of the crude reaction mixture.
Or
D.4. a compound of formula VIII 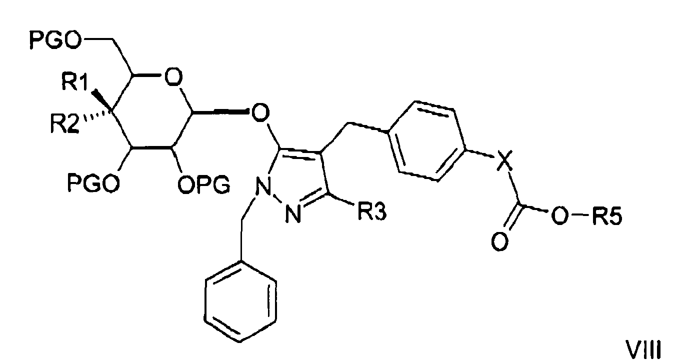
in which PG, X, R1, R2, R3 and R5 are as defined above;
is converted to the compound of the formula XIII 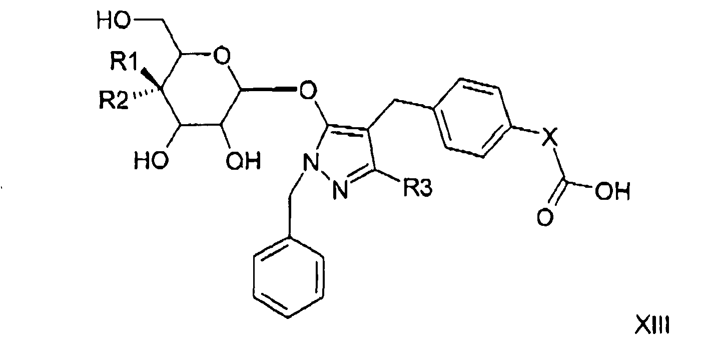
in which X, R1, R2 and R3 are as defined above;
by reaction with a base in an aqueous solvent at +10°C to +90°C,
and subsequent reacting of an solution or suspension of the compound of formula XIII with an excess of tris(hydroxymethyl)aminomethane in a suitable solvent with an amide forming reagent
to give a compound of formula XI 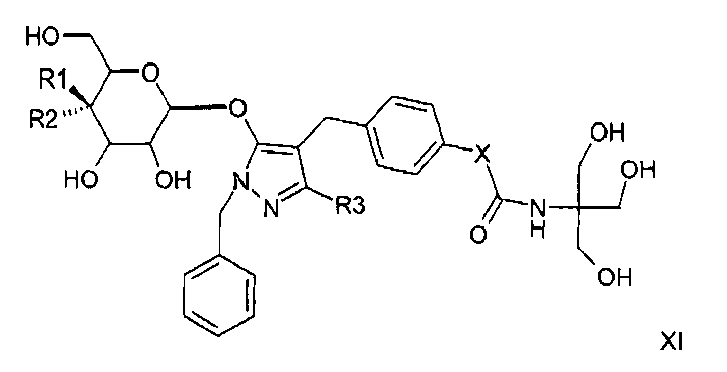
in which X, R1, R2 and R3 are as defined above;
and
subsequent hydrogenolysis with a heterogeneous platinum metal catalyst at a hydrogen pressure of 1 bar to 60 bars at 20°C to 90°C,
to give a compound of formula I 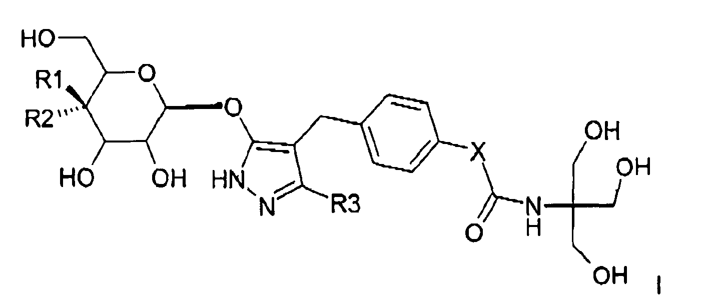
in which X, R1, R2 and R3 are as defined above;
and subsequently the compound of the formula (I) is purified by conventional purification methods such as crystallization or chromatography, preferably by crystallization from a solvent or a mixture of a plurality of solvents such as alkanes, aromatic compounds, halogenated solvents, ethers, ketones, esters, alcohols or water, particularly preferably by crystallization from alcohols or alcohols/water mixtures, very particularly preferably by crystallization from methanol/water.

4. A process for preparing a compound of the general formula (I) as claimed in claim 1, 2 or 3, wherein the compound of formula VII is 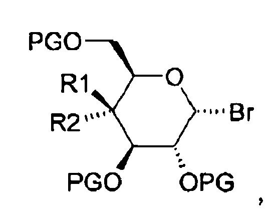 where
PG is benzoyl or acetyl;
R1 is H; and
R2 is F.

5. A process for preparing a compound of the general formula (I) as claimed in claim 1 to 4, in which
R1 is H;
R2 is F;
R3 is isopropyl;
R4, R5 are ethyl or methyl;
PG is benzoyl or acetyl;
X is (CH₂)₃.

6. The compound

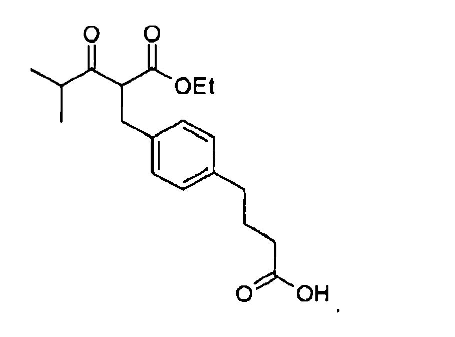

7. The compound

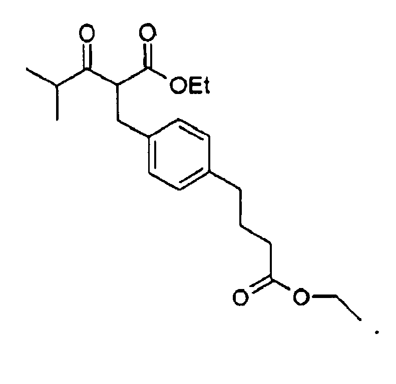

8. The compound

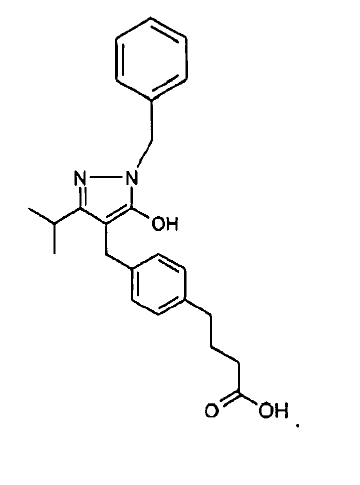

9. The compound

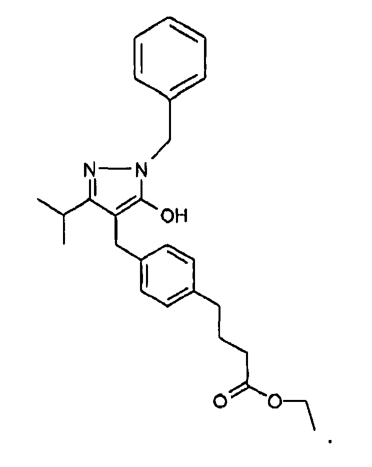

10. The compound 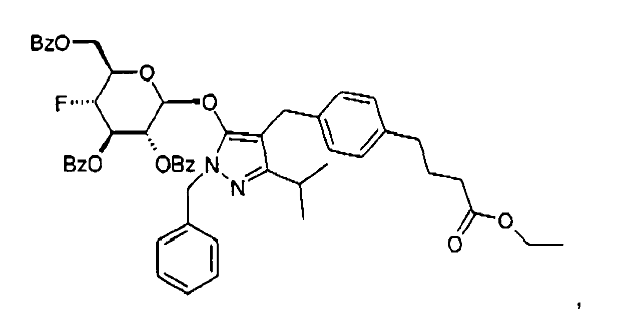 where Bz is benzoyl.

11. The compound 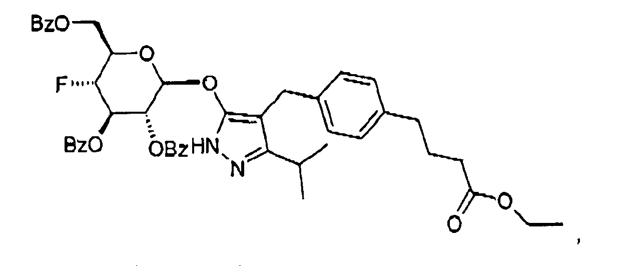 where Bz is benzoyl.

12. The compound

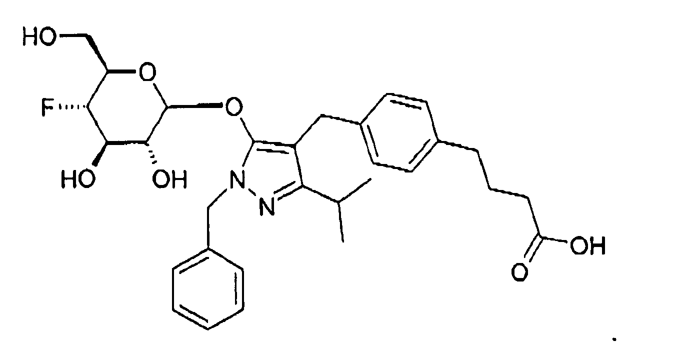

13. The compound
